# EUROPEAN PATENT APPLICATION

(11) **EP 1 811 383 A1**
(43) Date of publication of application: **25.07.2007**
(21) Application number: 05787558.5
(22) Date of filing: 28.09.2005
(51) Int. Cl.: G06F 12/00, G06F 17/30

(54) **ARRANGEMENT GENERATION METHOD AND ARRANGEMENT GENERATION PROGRAM**

(30) Priority: 01.10.2004 JP 2004290638
(71) Applicant: Turbo Data Laboratories, Inc., Yokohama-shi, Kanagawa 221-0005 (JP)
(72) Inventor: FURUSHO, Shinji, Court house Kikuna 804,, Yokohama-shi, Kanagawa 2210005 (JP)
(74) Representative: Zimmermann, Gerd Heinrich
(86) International application number: PCT/JP2005/017794
(87) International publication number: WO 2006/038498

(57) **Abstract**

A tree-type data structure representation method that can effectively trace relationships among data in a tree-type data structure, such as parent-child, ancestors, descendents, siblings, and generations, is provided. In a memory, data having a tree-type data structure in which unique node identifiers are assigned to nodes and a parent-child relationship between the nodes is represented by a C-P array including pairs, each pair being formed of a node identifier assigned to each of non-root nodes, which are nodes other than a root node, and a node identifier of a parent node with which each of the non-root nodes is associated is stored. In the memory, a vertex node list storing, in order to represent at least one node group, each including a specific node and a descendent node of the specific node, node identifiers of the specific nodes, which serve as vertex nodes, is also stored. A system 10 moves each of the vertex nodes to a child node, a parent node, or a node in the same generation as the vertex node (an older sibling node or a younger sibling node) by referring to the C-P array, and generates a new vertex node list.

## Description

### Technical Field

The present invention relates to a method for generating arrays representing a tree-type data structure, in particular, to a method for representing a tree-type data structure and constructing it on a storage device. The invention also relates to an information processing apparatus that employs the method. The invention further relates to a program executing the method.

### Background Art

Databases have been used for various purposes, and relational databases (RDBs), which can exclude logical inconsistencies, have been most commonly used for large- or intermediate-scale systems. RDBs are used for, e.g., airplane seat reservation systems. In this case, by specifying a key item, targets (in most cases, one target) can be quickly searched, or reservations can be confirmed, canceled, or changed. Since the number of seats in each flight is at most several hundred, the number of vacancies in a specific flight can also be determined.

It is known that RDBs are not suitable for handling tree-type data although they are suitable for handling table-format data (see, e.g., Non-Patent Document 1).

Additionally, some applications can be represented more appropriately by tree-type formats rather than table formats. In particular, XML using tree-type data structures, which serves as a data standard for intranet or Internet applications, has recently been widely used (see, e.g., Non-Patent Document 2 for details of XML).

Generally, however, the handling of tree-type data structures, e.g., the search for tree-type data, is very inefficient. The first reason for the inefficiency in handling tree-type data structures is that it is very difficult to specify locations of data promptly since data items are distributed in various nodes. In RDBs, data, e.g., "age", is stored only in an item named "age" of a certain table. In a tree-type data structure, however, since nodes storing data "age" are distributed in various locations, a target item of data cannot be searched unless the entire tree-type data structure is checked.

The second reason for the inefficiency in handling tree-type data structures is that the time required for representing search results is long. Representing a node group that is found by search often involves representation of descendent nodes of the node group. It takes a long time to represent descendent nodes since, unlike RDBMS, the tree-type data structures are of non-standard format.

Accordingly, to take advantage of RDBs, which are most commonly used as databases, a technique for converting tree-type data into an RDB when being converted into a database (see, e.g., Patent Document 1) has been proposed. In an RDB, data items are extracted and inserted into tables and are then stored as the tables. Accordingly, in order to convert actual tree-type data into an RDB, it is necessary to insert tree-type data into tables. In order to handle various tree-type data structures, system design should be conducted by means such as inserting into tables according to each structure. Thus, it is very time-consuming to construct a system based on RDBs.

On the other hand, a technique for converting tree-type data, in particular, XML data, into a database while keeping its original format has also been proposed. In the case of a tree-type data structure, since tree-type data can be represented in various manners, such as linking descendent nodes with one node, the time required for system design can be considerably reduced. Thus, there is now an increasing demand for processing tree-type data using means for handling a tree-type structure, such as XML.

An approach to converting XML data into a database while keeping its original format is to extract a copy of data input into a tree structure, and to separately store search index data for an item, for example, "age" (e.g., see Patent Document 2). This makes it possible to take full advantage of XML data, i.e., adding attributes to data itself, and also to store a relational structure of individual items represented by tags.
Patent Document 1: Japanese Unexamined Patent Application Publication No. 2003-248615
Patent Document 2: Japanese Unexamined Patent Application Publication No. 2001-195406
Non-Patent Document 1: SEC Co., Ltd. "Karearea White Paper", [online], [searched on February 19, 2004], Internet <URL:http://www.sec.co.jp/products/karearea/>
Non-Patent Document 2: "Extensible Markup Language (XML) 1.0 (Third Edition)", [online], February 4, 2004, [searched on February 19, 2004], Internet <URL:http://www.w3.org/TR/2004/REC-xml-20040204/>

### Disclosure of Invention

### Problems to be Solved by the Invention

According to an approach to separately storing search index data, however, data is stored at least doubly, and also, cost for creating an index is incurred and a data area for storing the index is required, which is disadvantageous in terms of storing a large amount of data.

Even if search is actually conducted to specify a node according to such a mechanism, it takes time to represent the node. Additionally, this mechanism cannot be used for conducting search involving a relationship between nodes (e.g., extracting a tree including "60" as "age" for ancestors and including "1" as "age" for descendents).

Such a basic problem of the related art originates from the following point. A tree-type data structure is represented by focusing on only each item of data and by then linking nodes that store the data therein by using pointers. Accordingly, the relationships between data items, such as parent-child, ancestors, descendents, brothers (siblings), or generations cannot be efficiently traced. In other words, since the values of the pointers are not fixed, they can be used only for representing storage addresses of data items, and cannot directly represent relationships between nodes.

Accordingly, it is an object of the present invention to provide a method for representing and constructing a tree-type data structure that allows efficient tracing of relationships between data items in the tree-type data structure.

It is another object of the present invention to provide an information processing apparatus used for constructing a tree-type data structure that allows efficient tracing of relationships between data items in the tree-type data structure.

It is another object of the present invention to provide a program used for representing and constructing a tree-type data structure that allows efficient tracing of relationships between data items in the tree-type data structure.

When handling a tree-type data structure, the necessity of moving a vertex node, which serves as a reference point, for following a location path, arises. It is thus another object of the present invention to provide a method, an information processing apparatus, and a program for moving a vertex node in a tree-type data structure.

### Means for Solving the Problems

The object of the present invention is achieved by an array generation method, in a computer including data having a tree-type data structure in which unique node identifiers are assigned to nodes and a parent-child relationship between the nodes is represented by a first array including pairs, each pair being formed of a node identifier assigned to each of non-root nodes, which are nodes other than a root node, and a node identifier of a parent node with which each of the non-root nodes is associated, the array generation method including: a step of providing a second array, in order to represent at least one node group, each including a specific node and a descendent node of the specific node, the second array storing node identifiers of the specific nodes, which serve as vertex nodes; and a step of generating a third array storing node identifiers of new vertex nodes, which are moved versions of the vertex nodes whose node identifiers are stored in the second array, by referring to the first array, wherein each of the vertex nodes is moved to one of a) a child node directly connected to the vertex node by an arc which is extended from the vertex node to the child node, b) a parent node directly connected to the vertex node by an arc which is extended from the parent node to the vertex node, c) an older sibling node which is in the same generation as the vertex node, an arc from the parent node of the vertex node being connected to the older sibling node before another arc from the parent node of the vertex node is connected to the vertex node, and d) a younger sibling node which is in the same generation as the vertex node, an arc from the parent node of the vertex node being connected to the younger sibling node after another arc from the parent node of the vertex node is connected to the vertex node.

In the present invention, in the new third array, the node identifiers of vertex nodes after being moved to one of a parent node, a child node, an older sibling node, or a younger sibling node are stored. This makes it possible to suitably change a reference point for following a location path, thereby facilitating, for example, tracing of data in a tree-type data structure.

In a preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to a child node may include a step of specifying, in the first array, a storage location at which the node identifier of the vertex node is stored and a step of determining a node identifier of a moved version of the vertex node to be a node identifier corresponding to the storage location.

In a preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to a parent node may include a step of specifying, in the first array, a node identifier stored at a location corresponding to the node identifier of the vertex node and a step of determining a node identifier of a moved version of the vertex node to be the node identifier stored at the corresponding location.

In another preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to an older sibling node may include a step of specifying, in the first array, a first node identifier stored at a storage location at which the node identifier of the vertex node is stored, a step of specifying, in the first array, a second node identifier stored at a storage location having a value smaller than a value of the location corresponding to the node identifier of the vertex node by one, and a step of determining, when the first node identifier and the second node identifier coincide with each other, a node identifier of a moved version of the vertex node to be a node identifier corresponding to the storage location at which the second node identifier is stored.

In still another preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to a younger sibling node may include a step of specifying, in the first array, a first node identifier stored at a location corresponding to the node identifier of the vertex node, a step of specifying, in the first array, a third node identifier stored at a storage location having a value greater than a value of the location corresponding to the node identifier of the vertex node by one, and a step of determining, when the first node identifier and the third node identifier coincide with each other, a node identifier of a moved version of the vertex node to be a node identifier corresponding to the storage location at which the third node identifier is stored.

In another preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to a child node may include a step of specifying, in the first array, a storage location at which the node identifier of the vertex node is stored and a step of determining a node identifier of a moved version of the vertex node to be a node identifier corresponding to the storage location.

In a preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to a parent node may include a step of specifying, in the first array, a node identifier stored at a location corresponding to the node identifier of the vertex node and a step of determining a node identifier of a moved version of the vertex node to be the node identifier stored at the corresponding location.

In another preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to an older sibling node may include a step of specifying, in the first array, a first node identifier stored at a storage location at which the node identifier of the vertex node is stored, a step of searching, in the first array, a fourth node identifier stored at storage locations having values smaller than a value of the storage location at which the node identifier of the vertex node is stored, the fourth identifier being equal to the first identifier, a step of specifying a storage location having a largest value among the storage locations of the fourth node identifier, and a step of determining a node identifier of a moved version of the vertex node to be a node identifier corresponding to the storage location having the largest value.

In another preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and the step of generating the third array for moving each of the vertex nodes to a younger sibling node may include a step of specifying, in the first array, a first node identifier stored at a storage location at which the node identifier of the vertex node is stored, a step of searching, in the first array, a fifth node identifier stored at storage locations having values greater than a value of the storage location at which the node identifier of the vertex node is stored, the fifth node identifier being equal to the first node identifier, a step of specifying a storage location having a smallest value among the storage locations of the fifth node identifier, and a step of determining a node identifier of a moved version of the vertex node to be a node identifier corresponding to the storage location having the largest value.

The object of the present invention can be achieved by an array generation program readable by a computer which includes data having a tree-type data structure, in which unique node identifiers are assigned to nodes and a parent-child relationship between the nodes is represented by a first array including pairs, each pair being formed of a node identifier assigned to each of non-root nodes, which are nodes other than a root node, and a node identifier of a parent node with which each of the non-root nodes is associated. The array generation program allows the computer to execute a step of providing a second array, in order to represent at least one node group, each including a specific node and a descendent node of the specific node, the second array storing node identifiers of the specific nodes, which serve as vertex nodes, and a step of generating a third array storing node identifiers of new vertex nodes, which are moved versions of the vertex nodes whose node identifiers are stored in the second array, by referring to the first array, wherein each of the vertex nodes is moved to one of a) a child node directly connected to the vertex node by an arc which is extended from the vertex node to the child node, b) a parent node directly connected to the vertex node by an arc which is extended from the parent node to the vertex node, c) an older sibling node which is in the same generation as the vertex node, an arc from the parent node of the vertex node being connected to the older sibling node before another arc from the parent node of the vertex node is connected to the vertex node, and d) a younger sibling node which is in the same generation as the vertex node, an arc from the parent node of the vertex node being connected to the younger sibling node after another arc from the parent node of the vertex node is connected to the vertex node.

In a preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to a child node, the program may allow the computer to execute a step of specifying, in the first array, a storage location at which the node identifier of the vertex node is stored and a step of determining a node identifier of a moved version of the vertex node to be a node identifier corresponding to the storage location.

In a preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to a parent node, the program may allow the computer to execute a step of specifying, in the first array, a node identifier stored at a location corresponding to the node identifier of the vertex node and a step of determining a node identifier of a moved version of the vertex node to be the node identifier stored at the corresponding location.

In another preferred embodiment, unique serial integers may be assigned to the nodes including the root node by giving priority to nodes in the same generation as a certain node rather than child nodes of that certain nodes, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to an older sibling node, the program may allow the computer to execute a step of specifying, in the first array, a first node identifier stored at a location corresponding to the node identifier of the vertex node, a step of specifying, in the first array, a second node identifier stored at a storage location having a value smaller than a value of the location corresponding to the node identifier of the vertex node by one, and a step of determining, when the first node identifier and the second node identifier coincide with each other, a node identifier of a moved version of the vertex node to be a node identifier corresponding to the storage location at which the second node identifier is stored.

In still another preferred embodiment, unique serial integers may be assigned to the nodes including the root node by giving priority to nodes in the same generation as a certain node rather than child nodes of that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to a younger sibling node, the program may allow the computer to execute a step of specifying, in the first array, a first node identifier stored at a location corresponding to the node identifier of the vertex node, a step of specifying, in the first array, a third node identifier stored at a storage location having a value greater than a value of the location corresponding to the node identifier of the vertex node by one, and a step of determining, when the first node identifier and the third node identifier coincide with each other, a node identifier of a moved version of the vertex node to be a node identifier corresponding to the storage location at which the third node identifier is stored.

In a preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to a child node, the program may allow the computer to execute a step of specifying, in the first array, a storage location at which the node identifier of the vertex node is stored and a step of determining a node identifier of a moved version of the vertex node to be a node identifier corresponding to the storage location.

In a preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to a parent node, the program may allow the computer to execute a step of specifying, in the first array, a node identifier stored at a location corresponding to the node identifier of the vertex node and a step of determining a node identifier of a moved version of the vertex node to be the node identifier stored at the corresponding location.

In another preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to an older sibling node, the program may allow the computer to execute a step of specifying, in the first array, a first node identifier stored at a storage location at which the node identifier of the vertex node is stored, a step of searching, in the first array, a fourth node identifier stored at storage locations having values smaller than a value of the storage location at which the node identifier of the vertex node is stored, the fourth identifier being equal to the first identifier, a step of specifying a storage location having a largest value among the storage locations of the fourth node identifier, and a step of determining a node identifier of a moved version of the vertex node to be a node identifier corresponding to the storage location having the largest value.

In still another preferred embodiment, unique serial integers may be assigned to the nodes including the root node by giving priority to child nodes of a certain node rather than nodes in the same generation as that certain node, the first array may be formed by arranging the integers assigned to the parent nodes of the corresponding non-root nodes, which are nodes other than the root node, according to an order in which the integers are assigned to the non-root nodes, and in the step of generating the third array for moving each of the vertex nodes to a younger sibling node, the program may allow the computer to execute a step of specifying, in the first array, a first node identifier stored at a storage location at which the node identifier of the vertex node is stored, a step of searching, in the first array, a fifth node identifier stored at storage locations having values greater than a value of the storage location at which the node identifier of the vertex node is stored, the fifth node identifier being equal to the first node identifier, a step of specifying a storage location having a smallest value among the storage locations of the fifth node identifier, and a step of determining a node identifier of a moved version of the vertex node to be a node identifier corresponding to the storage location having the largest value.

### Advantages

According to the present invention, a method for representing and constructing a tree-type data structure that allows efficient tracing of relationships between data items in the tree-type data structure can be provided.

According to the present invention, an information processing apparatus used for constructing a tree-type data structure that allows efficient tracing of relationships between data items in the tree-type data structure can be provided.

According to the present invention, a program used for representing and constructing a tree-type data structure that allows efficient tracing of relationships between data items in the tree-type data structure can be provided.

In particular, according to the present invention, a method, an information processing apparatus, and a program for generating and processing an array for representing at least one node group including a specific node and a descendent node of the specific node can be provided.

### Best Mode for Carrying Out the Invention

An embodiment of the present invention is described below with reference to the accompanying drawings.

### [Computer System Configuration]

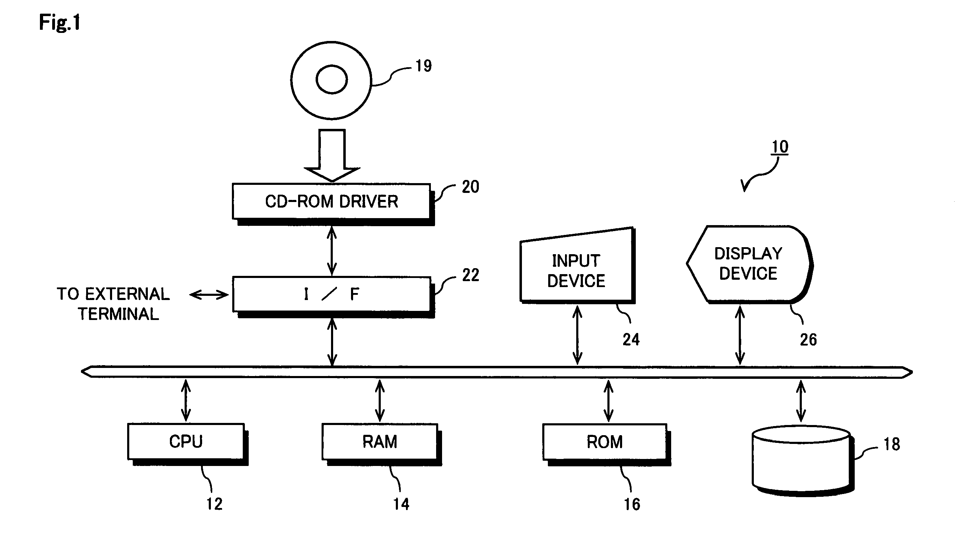

Fig. 1 is a block diagram illustrating the hardware configuration of a computer system that handles a tree-type data structure according to an embodiment of the present invention. The configuration of the computer system 10 is similar to that of a general computer system, as shown in Fig. 1, and includes a CPU 12 that controls the entire system and the individual elements by executing a program, a RAM (Random Access Memory) 14 that stores work data, etc., a ROM (Read Only Memory) 16 that stores programs, etc., a fixed storage medium 18, such as a hard disk, a CD-ROM driver 20 for accessing a CD-ROM 19, an interface (I/F) 22 disposed between the computer system 10 and the CD-ROM driver 20 or an external terminal connected to an external network (not shown), an input device 24, such as a keyboard and a mouse, and a CRT display device 26. The CPU 12, the RAM 14, the ROM 16, the external storage medium 18, the I/F 22, the input device 24, and the display device 26 are connected to each other with a bus 28 therebetween.

A program for constructing a tree-type data structure on a storage device and a program for converting the tree-type data structure on the storage device according to this embodiment may be stored in the CD-ROM 19 and are read by the CD-ROM driver 20, or may be stored in the ROM 16 beforehand. Alternatively, the programs read from the CD-ROM 19 may be stored in a predetermined area of the external storage medium 18. Alternatively, the programs may be supplied from an external source via a network (not shown), the external terminal, and the I/F 22.

An information processing apparatus according to an embodiment of the present invention can be implemented by allowing the computer system 10 to execute the program for constructing a tree-type data structure on a storage device and the program for converting the tree-type data structure on the storage device.

### [Tree-type Data Structure]

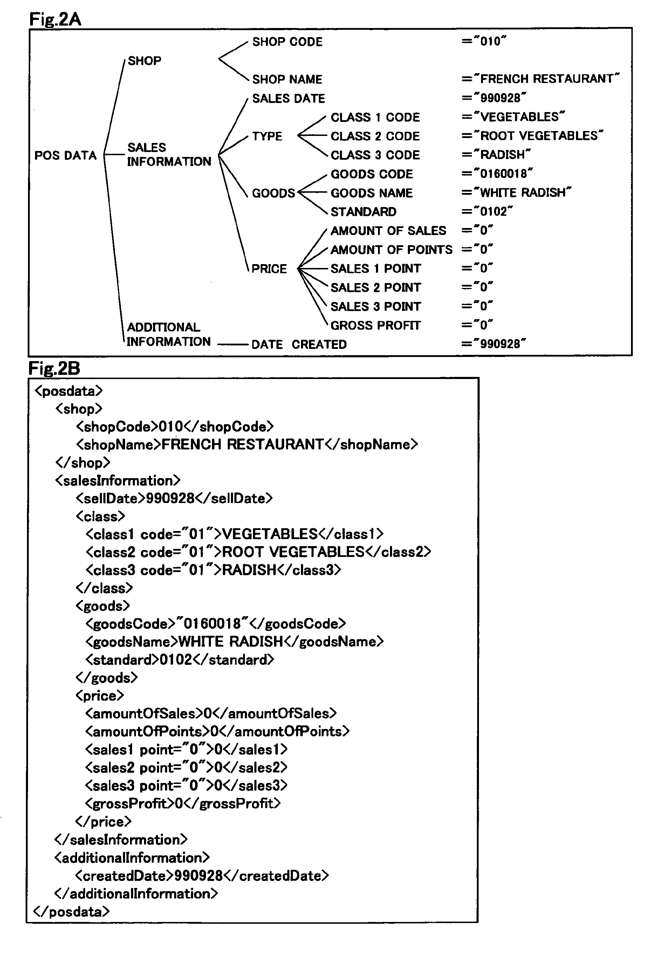

Figs. 2A and 2B illustrate POS data, which is an example of tree-type data. Fig. 2A illustrates an example of a visual representation of the data structure (i.e., topology) and the data values of this tree-type data. Fig. 2B illustrates an example of the same tree-type data represented in XML format. The tree-type data structure is represented, as shown in Figs. 2A and 2B, by combinations of nodes, starting from the root node (in this example, POS data) to the leaf nodes (endpoints) branched from corresponding nodes, and arcs. The substantial values of the nodes, e.g., the storage location of the value of a shop name node "French restaurant", is specified by the pointer related to the shop name node.

The present invention concerns the topology of a tree-type data structure. Accordingly, the topology of a tree-type data structure is mainly discussed below.

Conventionally, the above-described tree-type data structure is represented by linking nodes storing data therein by using pointers. Pointer representation, however, has a drawback, i.e., the lack of inevitability of pointer values. That is, the pointer values are not fixed for the same node. For example, in one case, a specific node A is stored in a certain address (e.g., 100), and in another case, the same node A is stored in another address (e.g., 200). Accordingly, the pointer values merely represent addresses at which the nodes are stored. Thus, if nodes are linked by using pointers according to the depth-first rule, it is difficult to re-link the nodes by using pointers according to the width-first rule.

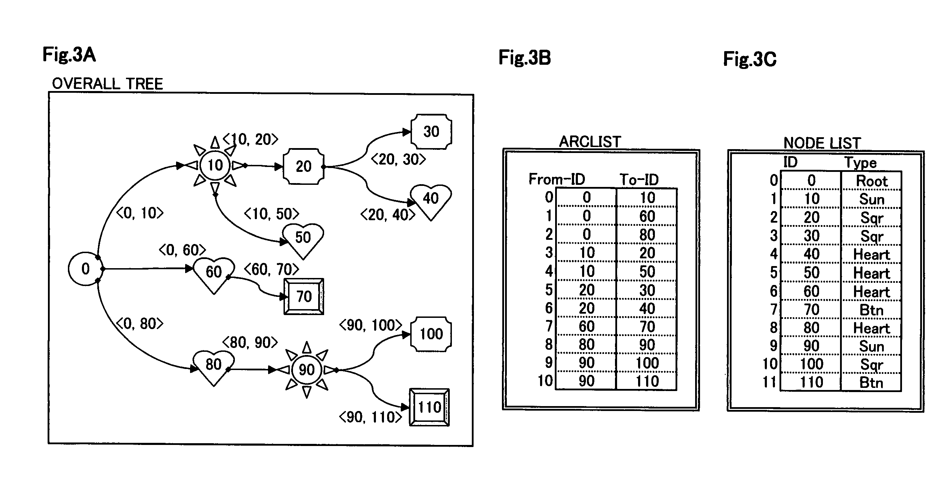

The present inventors have focused on the point that the topology of a tree-type data structure can be represented by an arclist. The arclist is a list of arcs representing a parent-child relationship among nodes. Figs. 3A through 3C illustrate one example of representation of a tree-type data structure using an arclist. In the example shown in Figs. 3A through 3C, a tree-type data structure consisting of 12 nodes to which node identifiers (IDs), such as 0, 10, 20, 30, 40, 50, 60, 70, 80, 90, 100, and 110, are assigned is shown. Fig. 3A illustrates the entire tree-type data structure. In Fig. 3A, the numbers shown at the centers of shapes, such as circles and heart-shaped figures, indicate node IDs, and a pair of numbers, such as <0, 10>, indicated next to an arrow connecting two shapes represent an arc. The node IDs are not restricted to character strings, and may be numerical values, in particular, integers. Fig. 3B illustrates an arclist from parent nodes (From-ID) to child nodes (To-ID), and Fig. 3C illustrates a node list including pairs, each including a node ID and a node type. To simply represent a tree-type data structure, the provision of a node list can be safely omitted. In principle, the use of such an arclist allows direct representation of relationships between nodes without using pointers.

### [Representation based on "Child→Parent" Relationship]

In the examples shown in Figs. 3A through 3C, the arclist is represented on the basis of a "parent→child" relationship for associating parent nodes with child nodes. Accordingly, since one parent node, for example, root node 0 is associated with three child nodes 10, 60, and 80, the same node ID 0 appears three times in From-ID of the arclist. That is, since a child node cannot be specified even if a parent node is specified, the arclist is formed of an element From-ID array and an element To-ID array. By the use of the arclist, a certain node appears both in the From-ID array and the To-ID array.

On the other hand, the parent-child relationship can also be represented by a "child→parent" relationship. In this case, the parent-child relationship between nodes is represented by an array consisting of pairs, each pair being formed of a non-root node, which is a node other than a root node, and an associated parent node. If the parent-child relationship is represented by the "child→parent" relationship, an important characteristic, which cannot be obtained by the "parent→child" relationship, is exhibited. That is, since one child node is always related to the unique parent node, if a child node is specified, the unique parent node related to that child node can be immediately specified. It is therefore sufficient to prepare only the element To-ID array for the arclist. As a result, the storage space required for storing the arclist can be reduced. A reduction in the storage space can also reduce the number of accesses to a memory, resulting in an acceleration of processing.

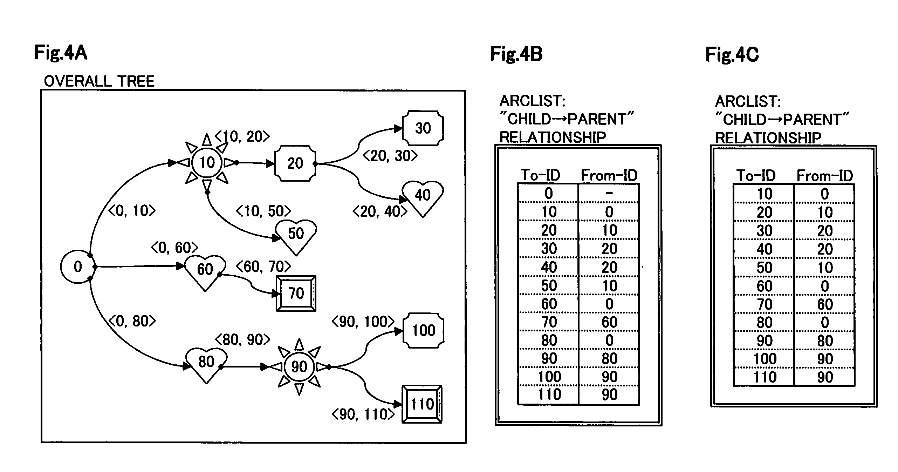

Figs. 4A through 4C illustrate a representation method for a tree-type data structure based on a "child→parent" relationship according to an embodiment of the present invention. Fig. 4A illustrates the overall tree and Fig. 4B illustrates an arclist based on the "child→parent" relationship. Since the arclist shown in Fig. 4B includes a parent-node storage area for the root node, "-" is set as the parent node of the root node for the sake of convenience. However, since the parent node related to the root node does not exist, the parent-node storage area for the root node can be excluded, as shown in Fig. 4C, from the arclist based on the "child→parent" relationship. In this manner, according to an embodiment of the present invention, by associating each non-root node, which is a node other than a root node, with a parent node of the non-root node, the parent-child relationship between nodes can be represented. Then, by following the list based on the "child→parent" representation from the child node to the parent node, the tree topology can be represented.

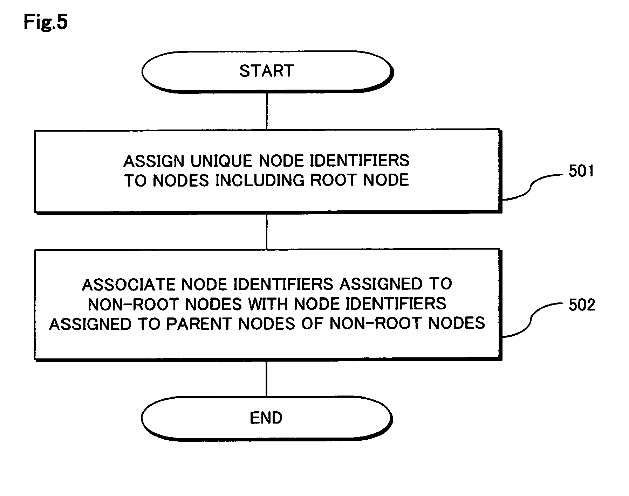

According to an embodiment of the present invention, the tree-type data structure based on the "child→parent" relationship is constructed on the RAM 14 by allowing, as shown in Fig. 5, the computer system 10 shown in Fig. 1 to execute a node definition step 501 of assigning unique node identifiers to nodes including the root node and a parent-child relationship definition step 502 of associating the node identifiers assigned to the non-root nodes, which are nodes other than the root node, with the node identifiers assigned to the parent nodes of the non-root nodes. In this manner, node identifiers are first assigned to nodes by the use of arbitrary identification information, such as character strings, floating points, or integers. Then, by the definition of the parent-child relationship based on the "child→parent" representation, the node identifiers of the parent nodes are derived (looked up) from the node identifiers of the child nodes. As a result, the tree topology can be represented.

### [Node Identifiers]

According to one preferable embodiment, in the node definition step, numerical values are used as node identifiers, and more preferably, serial integers are used, and even more preferably, serial integers starting from 0 or 1 are used. Accordingly, from the node identifiers, addresses at which the node identifiers of the parent nodes related to the corresponding child nodes are stored can be easily obtained. This makes it possible to increase the speed of the processing for looking up the node identifiers of the parent nodes from the node identifiers of the child nodes.

When representing a parent-child relationship between nodes by assigning ordered numbers to nodes in a tree-type data structure as node identifiers, the application of a rule to the order of assigning numbers facilitates the handling of the tree-type data structure. According to the present invention, as the rule applied to the order of assigning numbers, a depth-first mode in which priority is given to child nodes of a certain node rather than nodes in the same generation as that certain node and a width-first mode in which priority is given to nodes in the same generation as that certain node rather than child nodes of that certain node are used.

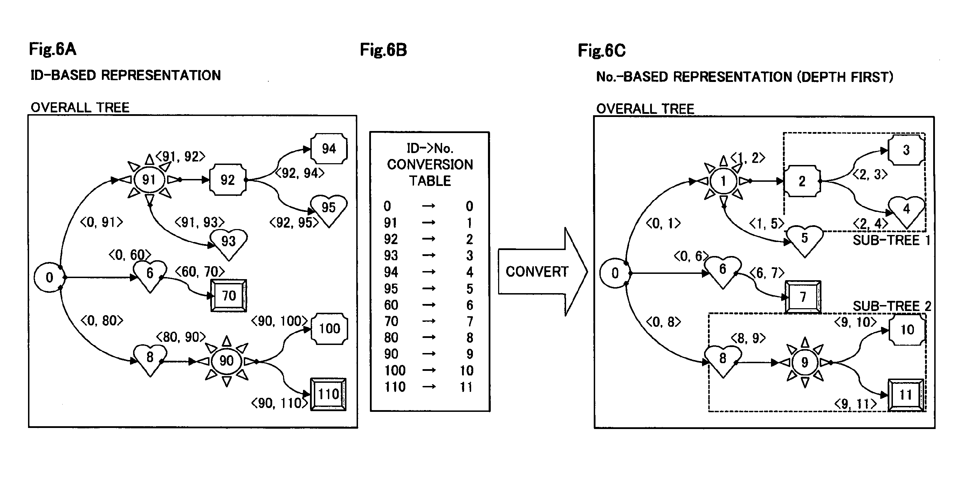

Figs. 6A through 6C illustrate processing for converting ID-based tree-structured data into serial-integer-based tree-structured data according to an embodiment of the present invention. Fig. 6A illustrates tree-structured data in which an ID number is assigned to each node. Fig. 6B illustrates a conversion rule. Fig. 6C illustrates tree-structured data in which a serial integer is assigned to each node. The conversion rule of this embodiment is a rule for assigning serial numbers in the depth-first mode, and more specifically, if there are a plurality of child nodes, the smallest number is assigned to the oldest child (oldest sibling), while the largest number is assigned to the youngest child (youngest sibling), and also, when assigning numbers, priority is given to child nodes rather than sibling nodes. Although in this embodiment numbers are assigned in ascending order, they may be assigned in descending order.

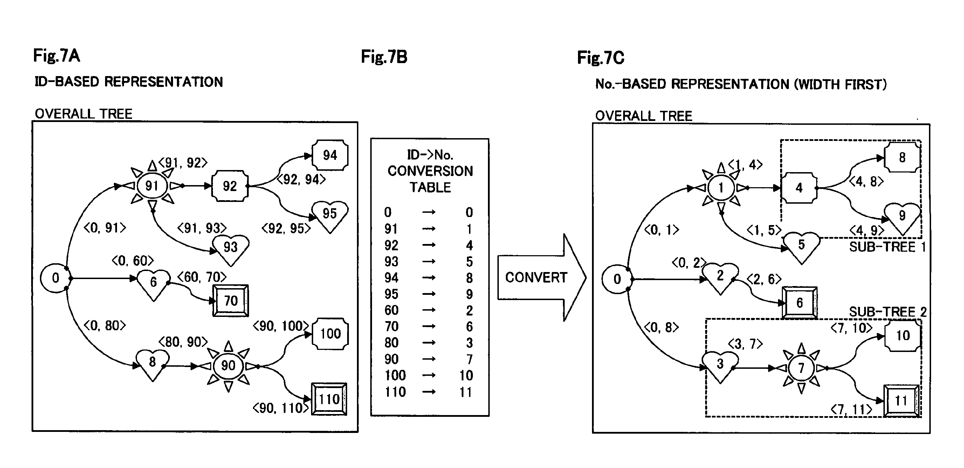

Figs. 7A through 7C illustrate processing for converting ID-based tree-structured data into serial-integer tree-structured data according to another embodiment of the present invention. Fig. 7A illustrates tree-structured data in which an ID number is assigned to each node. Fig. 7B illustrates a conversion rule. Fig. 7C illustrates tree-structured data in which a serial integer is assigned to each node. The conversion rule of this embodiment is a rule for assigning serial numbers in the width-first mode, and more specifically, if there are a plurality of child nodes, the smallest number is assigned to the oldest child (oldest sibling), while the largest number is assigned to the youngest child (youngest sibling), and also, when assigning numbers, priority is given to sibling nodes rather than child nodes. Although in this embodiment numbers are assigned in ascending order, they may be assigned in descending order.

The use of numbers as node identifiers in this manner makes it possible to look up, from a node number, promptly, i.e., in the order of O(1), the address at which the value of the node is stored. Additionally, by defining the parent-child relationship based on "child→parent" representation, the parent node can be looked up promptly, i.e., in the order of 0(1), from a child node.

### [Depth-first Mode]

According to an embodiment of the present invention, a depth-first tree-type data structure, such as that shown in Fig. 6C, can be constructed on a storage device by allowing the computer system 10 shown in Fig. 1 to execute a node definition step of assigning unique serial integers to nodes including the root node by giving priority to child nodes of a certain node rather than nodes in the same generation as that certain node and a parent-child relationship definition step of storing an array in which integers assigned to the parent nodes of the individual non-root nodes, which are nodes other than the root node, are arranged according to the order of the integers assigned to the non-root nodes. With this arrangement, serial integers are assigned to the nodes in the depth-first mode, and the parent-child relationship can be represented by an array based on "child→parent" representation.

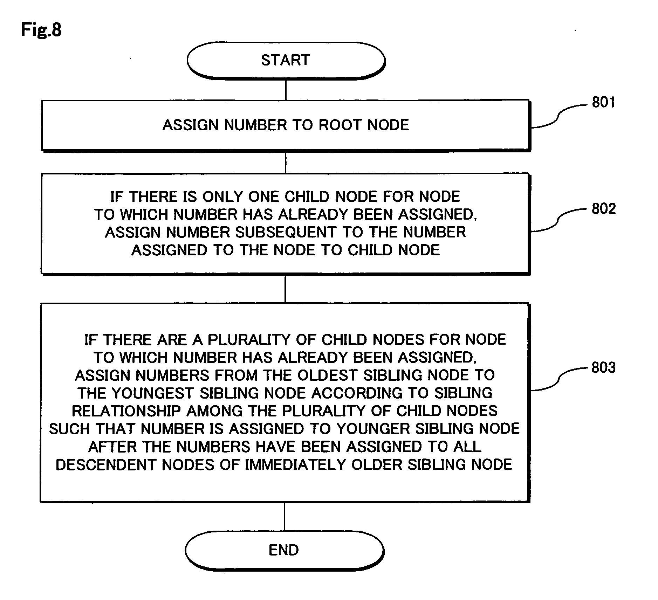

Fig. 8 is a flowchart illustrating depth-first-based node definition processing according to an embodiment of the present invention. This node definition processing allows the computer system 10 to execute a step 801 of assigning a number to the root node, a step 802 of assigning, if there is only one child node for a certain node to which a number has already been assigned, the number after the number assigned to the node to the child node, and a step 803 of assigning, if there are a plurality of child nodes for a certain node to which a number has already been assigned, numbers from the oldest sibling node to the youngest sibling node according to a sibling relationship among the plurality of child nodes such that a number is assigned to a younger sibling node after numbers have been assigned to all descendent nodes of the immediately older sibling node. With this arrangement, a sibling relationship can be defined among a plurality of child nodes that are branched off from the same parent node in the depth-first mode.

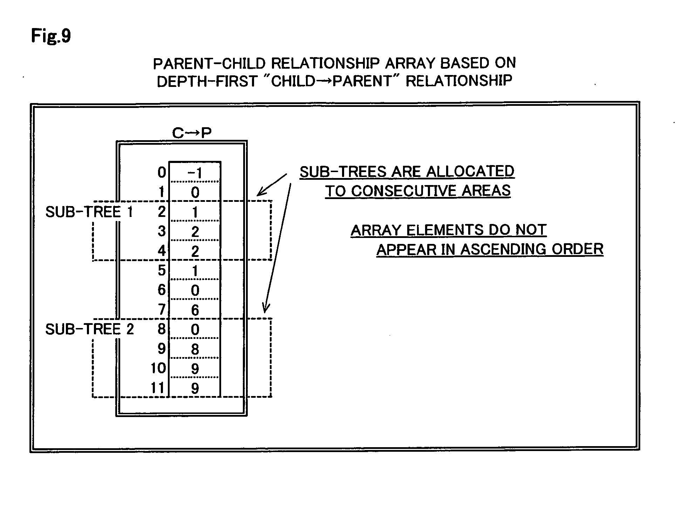

Fig. 9 illustrates a parent-child relationship array based on "child→parent" representation and created from the depth-first tree-type data structure shown in Fig. 6C according to an embodiment of the present invention. If the parent-child relationship of nodes to which serial numbers are assigned in the depth-first mode is represented as an array based on "child→parent" representation, as indicated by a sub-tree 1 or a sub-tree 2, as shown in Fig. 9, an excellent characteristic that descendent nodes of a certain node appear in consecutive locations can be obtained.

According to an embodiment of the present invention, by utilizing the excellent characteristic of the depth-first mode, all descendent nodes of a certain node can be specified by extracting consecutive locations in which integers greater than the integer assigned to the certain node are stored from the above-described array. Accordingly, a node group indicating descendent nodes of a certain node can be obtained as a continuous block in the array. For example, if the size of the continuous block is m, the processing speed for specifying all descendent nodes of a certain node is on the order of O(m) .

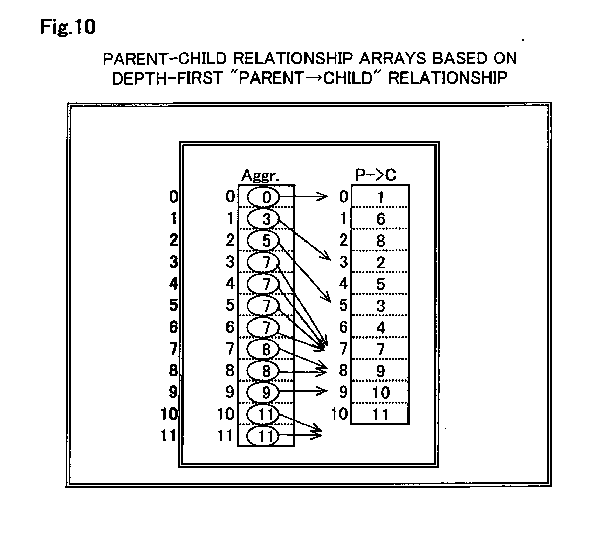

As discussed above, the parent-child relationship can be represented by, not only an array based on "child→parent" representation, but also an array based on "parent→child" representation. Fig. 10 illustrates a parent-child relationship array based on "parent→child" representation and created from the depth-first tree-type data structure shown in Fig. 6C. Since there are a plurality of child nodes for one parent node, two parent-child-relationship arrays are required: an array Aggr for indicating areas in which the numbers assigned to child nodes of each node are stored and the other array P→C in which the numbers assigned to the child nodes are stored. For example, the value of the second element Aggr[1] from the head of the array Aggr is "3", which means that the numbers assigned to child nodes for node[1] are stored after the element P→C[3] of the array P→C. It can thus seen that the child nodes of node[0], i.e., the root node, are three elements from the head of the array P→C, such as 1, 6, and 8 associated with P→C[0], P→C[1], and P→C[2], respectively.

A process for determining parent-child relationship arrays based on "parent→child" representation is discussed below.
(1) If the number assigned to a node coincides with the largest index (=11) of array P→C, no child node exists for this node. Accordingly, processing is discontinued.
(2) The Aggr value is determined from the number assigned to a parent node indicated in bold face in Fig. 10. The Aggr value represents the start point of array P→C.
(3) The Aggr value obtained by adding one to the number assigned to the parent node is determined. The value obtained by subtracting one from the Aggr value is the end point of array P→C.

For example, the start point of the child nodes of node 0 is Aggr[0], i.e., 0, and the end point is Aggr[1]-1, i.e., 3-1=2. Accordingly, the child nodes of node 0 are the zero-th through the second elements of array P→C, i.e., 1, 6, and 8.

Alternatively, the parent-child relationship based on "parent→child" representation can be more simply represented, i.e., by two arrays, such as an array of parent node numbers and an array of child node numbers. To determine the parent-child relationship by utilizing those arrays, however, the parent node numbers should be searched, i.e., an access time log(n) is required, which is inefficient.

### [Width-first Mode]

A width-first-based tree-type data structure, such as that shown in Fig. 7C, can be constructed on a storage device by allowing the computer system 10 shown in Fig. 1 to execute a node definition step of assigning unique serial integers to nodes including the root node by giving priority to nodes in the same generation as a certain node rather than child nodes of that certain node and a parent-child relationship definition step of storing, in the storage device, an array in which integers assigned to the parent nodes of non-root nodes, which are nodes other than the root nodes, are arranged according to the order of the integers assigned to the non-root nodes. With this arrangement, serial numbers are assigned to the nodes in the width-first mode, and the parent-child relationship between nodes can be represented by an array based on "child→parent" relationship.

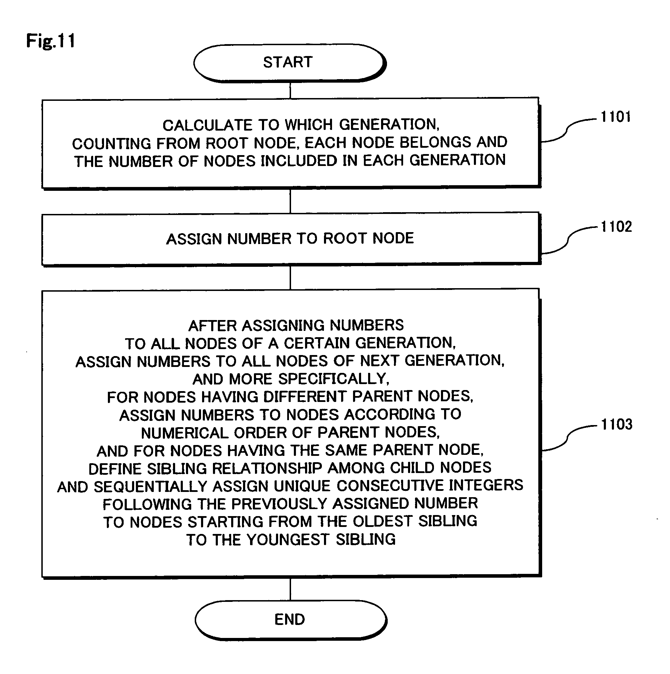

Fig. 11 is a flowchart illustrating width-first-based node definition processing according to an embodiment of the present invention. This node definition step allows the computer system 10 to execute a step 1101 of calculating to which generation, counting from the root node, each node belongs and the number of nodes included in each generation, a step 1102 of assigning a number to the root node, and a step 1013 of assigning, after assigning numbers to all nodes included in a certain generation, numbers to all nodes included in the next generation, and more specifically, for nodes having different parent nodes, assigning numbers to the nodes according to the order of the numbers assigned to the parent nodes, and for nodes having the same parent node, defining the sibling relationship among a plurality of child nodes branched off from the parent node and assigning unique serial integers following the previously assigned number to nodes starting from the oldest sibling node to the youngest sibling node. With this arrangement, the sibling relationship can be defined among a plurality of child nodes branched off from the same parent node in the width-first mode.

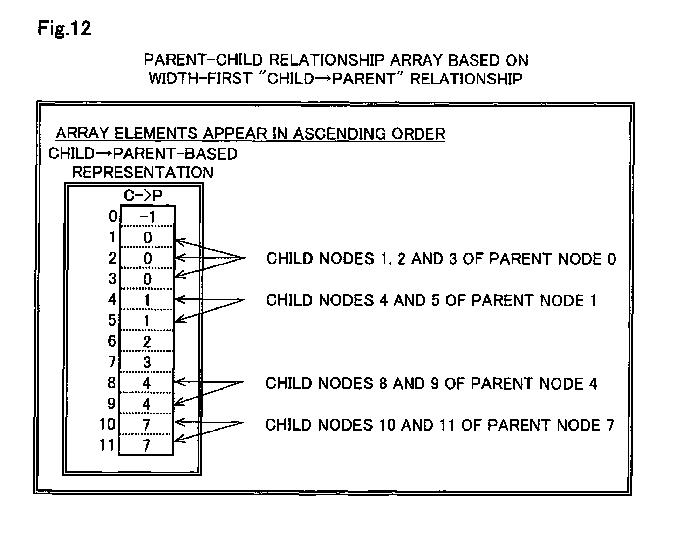

Fig. 12 illustrates a parent-child relationship array based on "child→parent" representation and created from the width-first tree-type data structure shown in Fig. 7C according to an embodiment of the present invention. If the parent-child relationship of nodes to which serial numbers are assigned in the width-first mode is represented as an array based on "child→parent" representation, as indicated by Fig. 12, an excellent characteristic that child nodes of a certain node appear in consecutive locations can be obtained. The reason for this is that, if the parent-child relationship among nodes to which serial numbers are assigned in the width-first mode is represented by an array based on "child→parent" representation, the numbers assigned to parent nodes appear in the array in a certain order (ascending or descending order).

According to an embodiment of the present invention, by utilizing the excellent characteristic of the width-first mode, all child nodes of a certain node can be specified by extracting consecutive locations in which the same value as the integer assigned to the certain node is stored from the above-described array. Accordingly, child nodes of a certain node can be searched according to a binary search technique, i.e., in the order of O(log(n)).

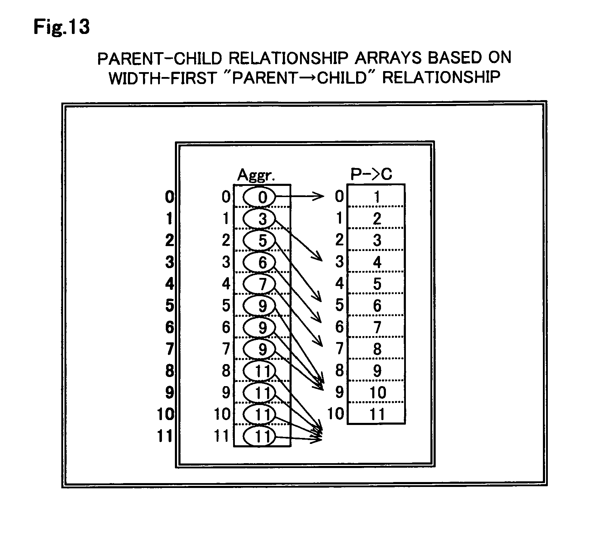

As discussed above, the parent-child relationship can be represented, not only by an array based on "child→parent" representation, but also by an array based on "parent→child" representation. Fig. 13 illustrates a parent-child relationship array based on "parent→child" representation and created from the width-first tree-type data structure shown in Fig. 7C. Since there are a plurality of child nodes for one parent node, two parent-child-relationship arrays are required: an array Aggr for indicating areas in which the numbers assigned to child nodes of each node are stored and the other array P→C in which the numbers assigned to the child nodes are stored. For example, the value of the second element Aggr[1] from the head of the array Aggr is "3", which means that the numbers assigned to child nodes for node[1] are stored after the element P→C[3] of the array P→C. It can thus be seen that the child nodes of node[0], i.e., the root node, are three elements from the head of the array P→C, such as 1, 2, and 3 associated with P→C[0], P→C[1], and P→C[2], respectively.

A process for determining parent-child relationship arrays based on parent→child representation is discussed below.
(1) If the number assigned to a node coincides with the largest index (=11) of array P→C, no child node exists for this node. Accordingly, processing is discontinued.
(2) The Aggr value is determined from the number assigned to a parent node indicated in bold face in Fig. 13. The Aggr value represents the start point of array P→C.
(3) The Aggr value obtained by adding one to the number assigned to the parent node is determined. The value obtained by subtracting one from the Aggr value is the end point of array P→C.

For example, the start point of the child nodes of node 0 is Aggr[0], i.e., 0, and the end point is Aggr[1]-1, i.e., 3-1=2. Accordingly, the child nodes of node 0 are the zero-th through the second elements of array P→C, i.e., 1, 2, and 3.

### [Vertex Nodes and Partial Tree Group]

Representing, in the above-described tree, all nodes starting from a node closest to the root node to the leaf node (endpoint) branched off from the node is now considered. A node group from a certain node to the leaf node is referred to as a "partial tree". The node closest to the above-described node (root node) is referred to as a "vertex node".

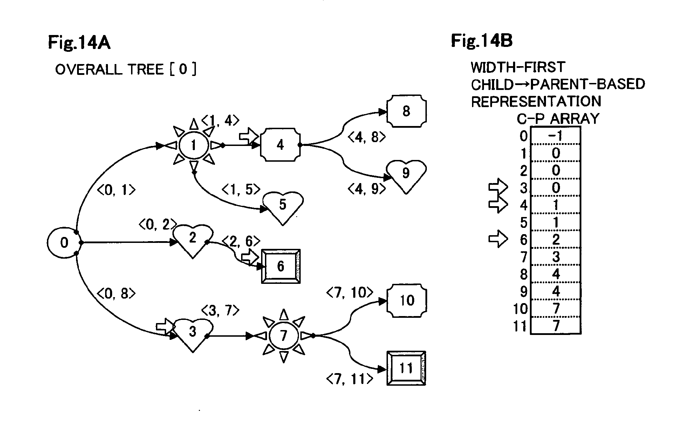

Fig. 14A illustrates a width-first-based tree-type data structure, and Fig. 14B illustrates a parent-child relationship array based on "child→parent" representation. For example, a vertex node [4] includes node identifiers {4, 8, 9}, a vertex node [6] includes a node identifier {6}, and a vertex node [3] includes node identifiers {3, 7, 10, 11}. An array including a plurality of vertex nodes is referred to as a "vertex node list". According to a vertex node list, a plurality of partial trees can be specified, and the specified plurality of partial trees are referred to as a "partial tree group".

The vertex node list is represented by [a, b, ...], where "a", "b", ... indicate node identifiers related to the vertex nodes. It is now considered that, by developing each vertex node forming the vertex node list, the node identifiers of all nodes contained in a partial tree having the vertex node is determined. In a list of the determined node identifiers, if a node identifier appears only once, i.e., if the same node identifier does not appear more than once, such a partial tree group is referred to as a "normalized partial tree group", and partial tree groups other than normalized partial tree groups are referred to as "non-normalized partial tree groups".

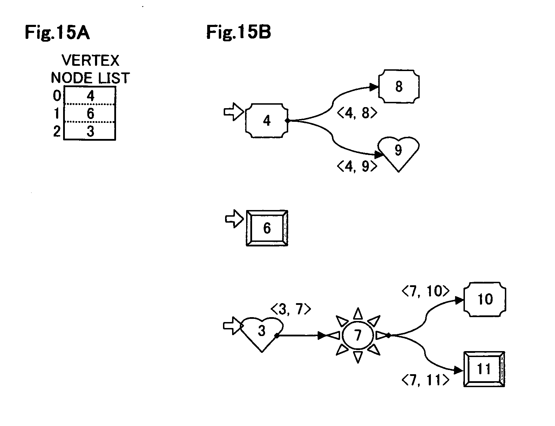

Regardless of normalized partial tree groups or non-normalized partial tree groups, from a vertex node list, a partial tree group including vertex nodes and descendent nodes thereof can be specified. For example, from a vertex node list [4, 6, 3] shown in Fig. 15A, a partial tree group (partial trees {4, 8, 9}, {6}, {3, 7, 10, 11}) shown in Fig. 15B can be specified.

The partial tree group specified by a vertex node list can be subjected to search, counting, sorting, and set operations.

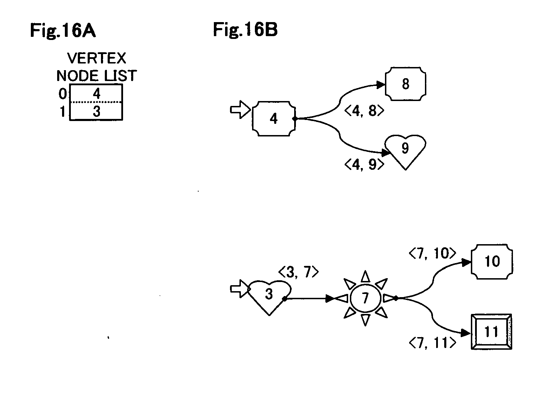

In the example shown in Figs. 15A and 15B, for example, if partial trees including heart-shaped figures are searched, a partial tree group shown in Fig. 16B can be obtained. Fig. 16A illustrates a vertex node list representing this partial tree group.

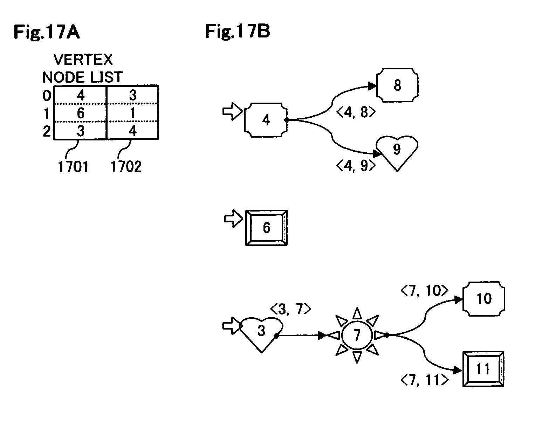

If the number of nodes belonging to each partial tree is counted, the result of counting can be shown as in Fig. 17B. In Fig. 17A, an array 1701 indicates a vertex node list, and an array 1702 indicates the number of nodes belonging to the partial tree specified by each vertex node.

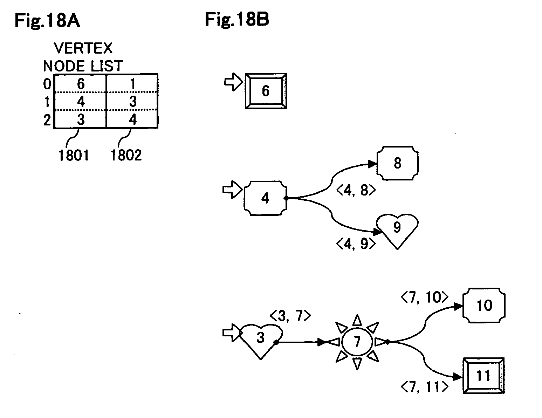

As a sorting operation, sorting partial trees by the numbers of nodes belonging to the partial trees can be considered. In Fig. 18A, an array 1801 indicates a sorted vertex node list, and an array 1802 indicates the numbers of nodes belonging to the partial trees specified by the vertex node list. Fig. 18B illustrates the state in which the partial trees are sorted by the number of nodes.

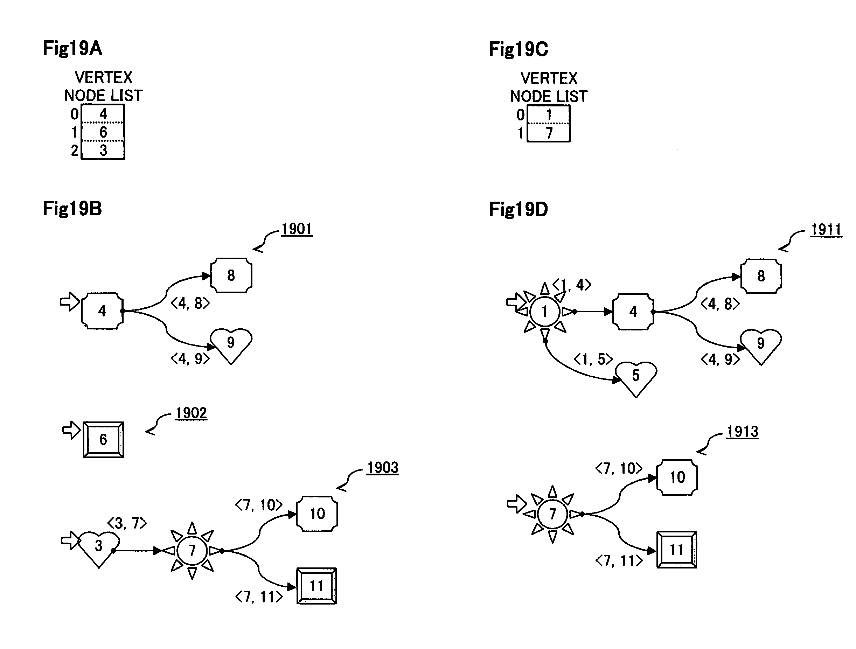

As a set operation between a plurality of partial tree groups, a logical AND is now considered. In the tree shown in Figs. 14A and 14B, the logical AND of the partial tree group shown in Fig. 19B (the corresponding vertex node list is shown in Fig. 19A) and the partial tree group shown in Fig. 19D (the corresponding vertex node list is shown in Fig. 19C) is now considered.

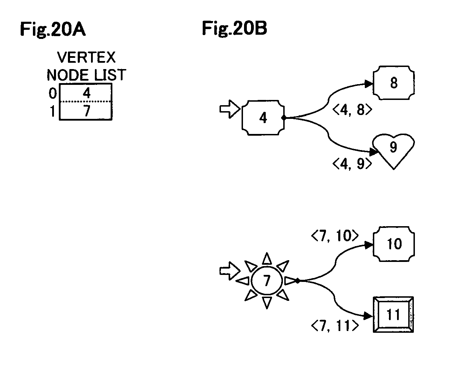

Upon comparing a partial tree 1901 specified by the vertex node having the node identifier [4] shown in Fig. 19B with a partial tree 1911 specified by the vertex node having the node identifier [1] shown in Fig. 19D, the partial tree 1901 is included in the partial tree 1902. In the partial tree group shown in Fig. 19D, there is no partial tree that includes or is included in the partial tree 1902 shown in Fig. 19B. Upon comparing a partial tree 1903 specified by the vertex node having the node identifier [3] in Fig. 19B with a partial tree 1913 specified by the node identifier [7] shown in Fig. 19D, the partial tree 1913 is included in the partial tree 1903. As a result, the vertex node list indicating the result of executing the logical AND can be represented by [4, 7], as shown in Fig. 20A. Fig. 20B illustrates a partial tree group corresponding to the result of the logical AND.

As is seen from Figs. 16A through 20B, from the vertex node lists (for a counting operation, in addition to the vertex node list, an array, which has the same size as the vertex node list, storing a counting result (the number of nodes)), the results of the corresponding processing or operations can be represented.

### [Movement of Vertex Node]

In table-format data, because of the regular arrangement of items, an operation for specifying a cell (or a column or a row) to be displayed or edited is easy. In contrast, in tree data, because of the irregular arrangement of nodes, an operation for specifying a node (corresponding to a "cell" in table-format data) group to be displayed, edited, or counted becomes essential. The above-described vertex node makes it possible to specify a node group to be displayed, edited, or counted. The node that specifies a node group to be displayed, edited, or counted may be referred to as a "context node". In this specification, therefore, the vertex node has the same function as the context node.

In the above-described operations, such as search, counting, sorting, and set operations, a new value different from the values in the vertex node list does not appear. In the operation performed on partial tree groups, however, the necessity of moving the topology of a tree often arises.

A tree representing a family structure having a parent as a vertex node, for example, is now considered. Currently, the vertex node is located at a mother node. To obtain a list of all children, however, it may be necessary to move the vertex node from the mother node to a child node. A vertex node list of a normalized partial tree group does not necessarily remain as a vertex node list of a normalized partial tree group, and may become a vertex node list of a non-normalized partial tree group, after the vertex node is moved.

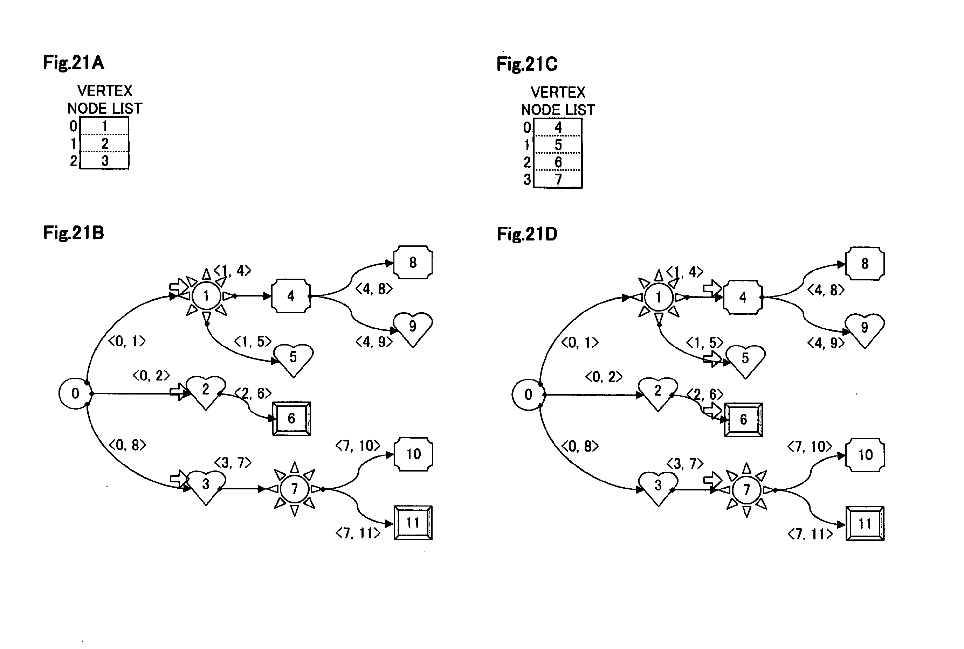

An example of moving a vertex node is discussed below. In the tree shown in Fig. 21B, nodes having the node identifiers "1", "2", and "3" are vertex nodes, as indicated by the vertex node list in Fig. 21A, and then, the case where the vertex nodes are moved to nodes corresponding to "children" is now considered. In Figs. 21B and 21D, nodes pointed by the arrows are vertex nodes.

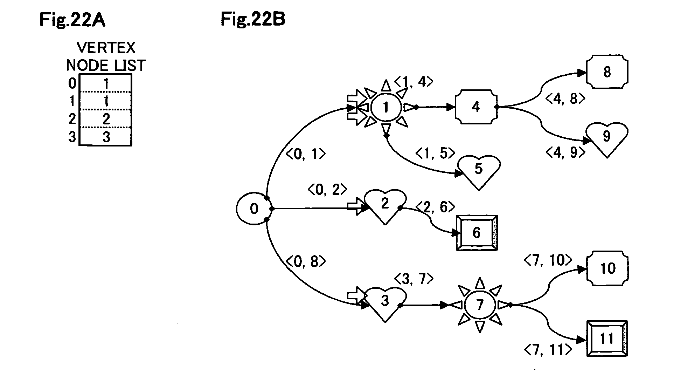

In this case, as shown in Fig. 22D, the vertex node having the node identifier "1" is moved to nodes having the node identifiers "4" and "5", the vertex node having the node identifier "2" is moved to a node having the node identifier "6", and the vertex node having the node identifier "3" is moved to a node having the node identifier "7". As a result, after the vertex nodes have been moved, the vertex node list representing the vertex nodes can be represented by, as shown in Fig. 21C, [4, 5, 6, 7].

Suppose vertex nodes will be moved to nodes corresponding to "parents" when the nodes having the node identifiers "4", "5", "6", and "7" are vertex nodes, as shown in Fig. 21D. The vertex node having the node identifier "4" and the vertex node having the node identifier "5" are moved to the node having the node identifier "1". The vertex node having the node identifier "6" is moved to the node having the node identifier "2". The vertex node having the node identifier "7" is moved to the node having the node identifier "3". As a result, the vertex node list representing the vertex nodes after the vertex nodes are moved can be represented by [1, 1, 2, 3].

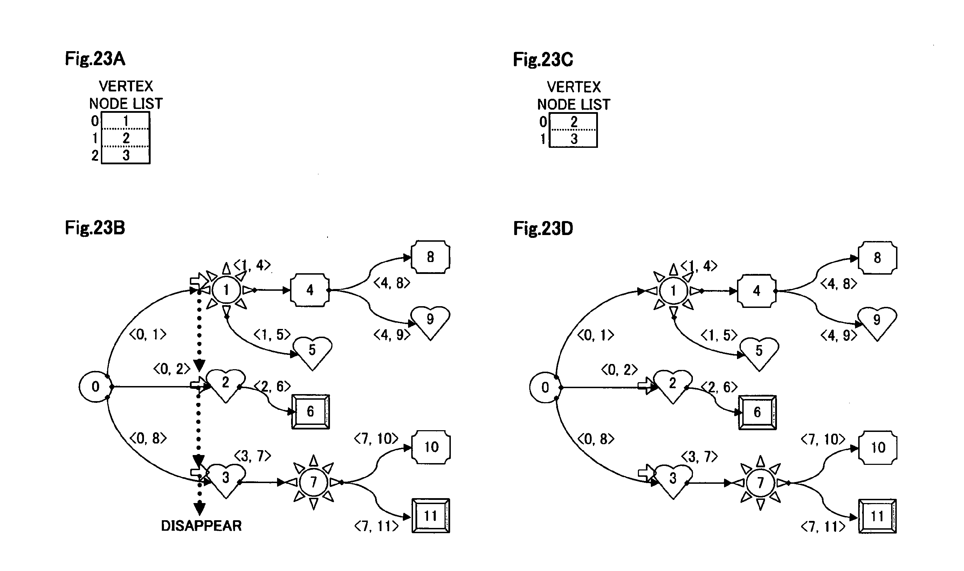

Then, it is now considered that each of vertex nodes having the node identifiers "1", "2", and "3", as shown in Fig. 23B, is moved to a node which is in the same generation and which is the immediate "younger sibling" node. That is, according to the rule for assigning node identifiers in the present invention, among nodes having the same parent node and being in the same generation, moving each of the vertex nodes to the node having the node identifier closest to the node identifier of the vertex node is now considered (see the broken arrows in Fig. 23B). A "younger sibling" node is a node in the same generation as a vertex node and is a node to which an arc from the parent node of the vertex node is connected after another arc from the parent node is connected to the vertex node. An "older sibling" node is a node in the same generation as a vertex node and is a node to which an arc from the parent node of the vertex node is connected before another arc from the parent node is connected to the vertex node.

As shown in Figs. 23B and 23D, the vertex node having the node identifier "1" is moved to the node having the node identifier "2", and the vertex node having the node identifier "2" is moved to the node having the node identifier "3". On the other hand, the vertex node having the node identifier "3" disappears since there is no "younger sibling" node for this vertex node. Accordingly, the vertex node list representing vertex nodes after the vertex nodes are moved can be represented by [2, 3], as shown in Fig. 23C.

### [Processing Executed when Moving Vertex Nodes (Width-first Mode)]

Processing executed when moving a vertex node according to an embodiment of the present invention is discussed below. A description is first given of the movement of vertex nodes when an array (C-P array) based on "child→parent" representation created from a tree-type data structure based on a width-first mode is used.

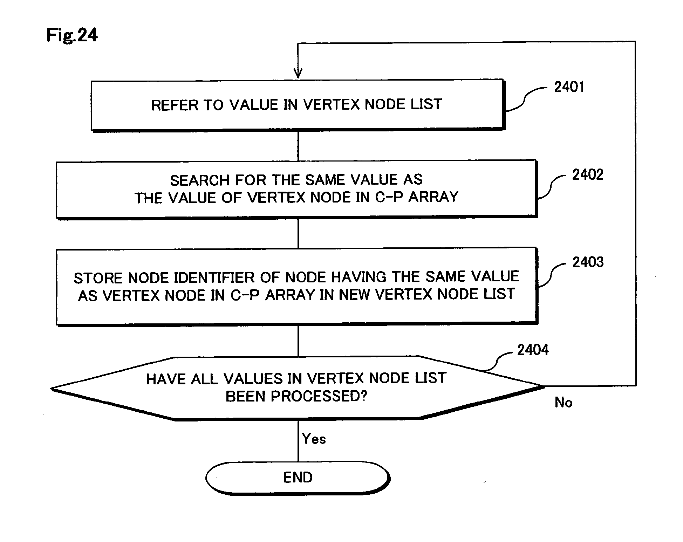

Fig. 24 is a flowchart illustrating processing executed by the computer system 10 when moving a vertex node to a node corresponding to a child. As shown in Fig. 24, the computer system 10 refers to a value in a vertex node list in which the node identifiers of vertex nodes are stored (step 2401), and searches for the same value in the C-P array as the value (node identifier) of the vertex node list (step 2402). Then, the computer system 10 stores the node identifiers of the nodes in the C-P array having the same value as the node identifier of the vertex node in a new vertex node list (step 2403). Steps 2401 through 2403 are executed on all the values in the vertex node list (see step 2404), and then, the node identifiers of the new vertex nodes corresponding to children are stored in the new vertex node list.

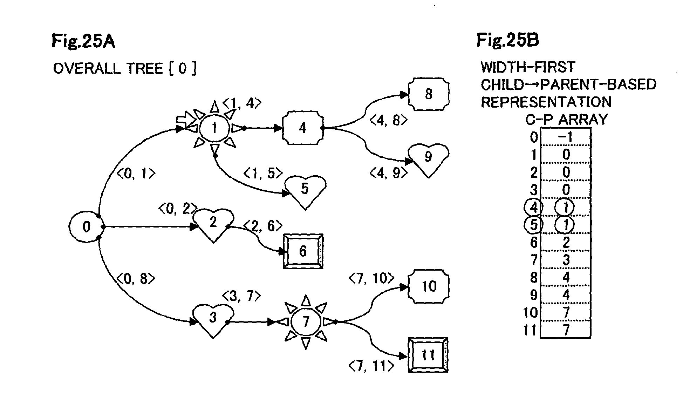

It is now assumed that, in the example shown in Fig. 25A, the vertex node list is represented by [1, 2, 3]. The vertex node having the node identifier "1" (see the arrow) is now focused on. Then, as a result of searching the C-P array, it can be seen that the values in the C-P array of the node identifiers "4" and "5" are the same as the value "1" in the vertex node list (see Fig. 25B). Then, in a new vertex node list, the values "4" and "5" are stored. Since the values are assigned in ascending order in a width-first-mode C-P array, search in step 2402 is easy. As a result of executing processing similar to the above-described processing on the other values "2" and "3" in the vertex node list, a new vertex node list [4, 5, 6, 7] can be obtained.

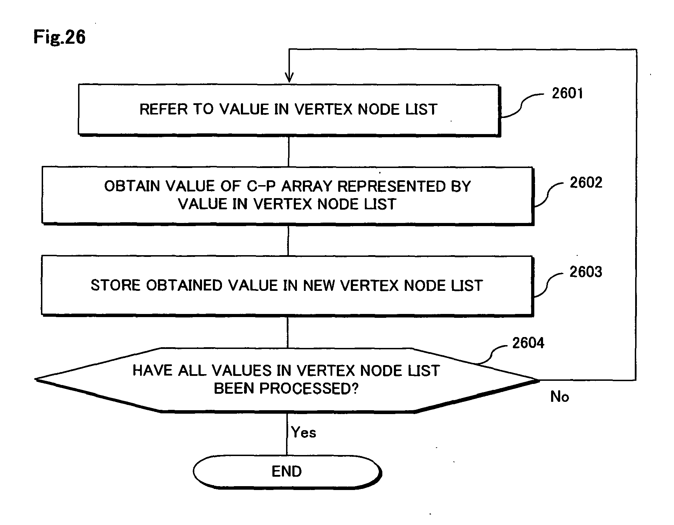

Fig. 26 is a flowchart illustrating processing executed by the computer system 10 when moving a vertex node to a node corresponding to a parent (parent node). As shown in Fig. 26, the computer system 10 refers to a value in a vertex node list (step 2601), and obtains a value in the C-P array indicated by the value of the vertex node list (step 2602). The computer system 10 then stores the obtained value in a new vertex node list (step 2603). Steps 2601 through 2603 are executed on all the values in the vertex node list (see step 2604), and then, the node identifiers of the new vertex nodes corresponding to parents are stored in the new vertex node list.

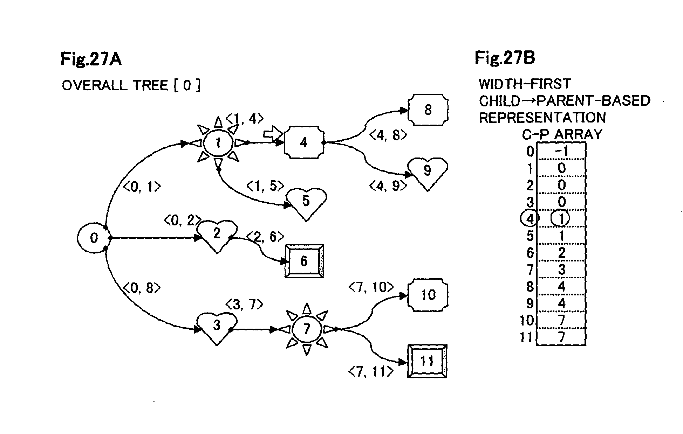

It is now assumed that, in Fig. 27A, the vertex node list is represented by [4, 5, 6, 7]. The vertex node having the node identifier "4" (see the arrow) is now focused on. Then, it can be seen that the value corresponding to the node identifier "4" in the C-P array is "1", and then, the value "1" is stored in a new vertex node list. Similarly, the values in the C-P array corresponding to the node identifiers "5", "6", and "7" of the other nodes stored in the vertex node list are "1", "2", and "3", respectively. Accordingly, the new vertex node list results in [1, 1, 2, 3].

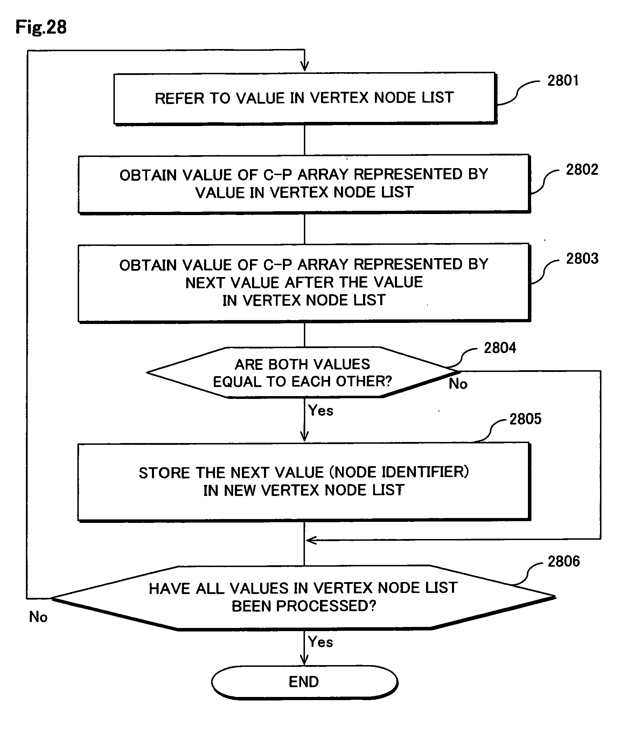

Fig. 28 is a flowchart illustrating processing executed by the computer system 10 when moving a vertex node to a node corresponding to a younger sibling (younger sibling node). As shown in Fig. 28, the computer system 10 refers to a value in a vertex node list (step 2801), and obtains a value (node identifier) in the C-P array indicated by the value of the vertex node list (step 2802). Then, the computer system 10 obtains the value (node identifier) in the C-P array indicated by the next value (in this embodiment, the value obtained by adding "1" to the previous value) of the vertex node (step 2803).

Then, the computer system 10 compares the obtained two values, and if both values coincide with each other (YES in step 2804), the computer system 10 stores the above-described next value (node identifier) in a new vertex node list (step 2805). On the other hand, if both values do not coincide with each other, it is determined that the vertex node disappears if it is moved.

The computer system 10 executes steps 2601 through 2603 on all the values in the vertex node list (see step 2806), and then, the node identifiers of the new vertex node corresponding to younger sibling nodes are stored in the new vertex node list.

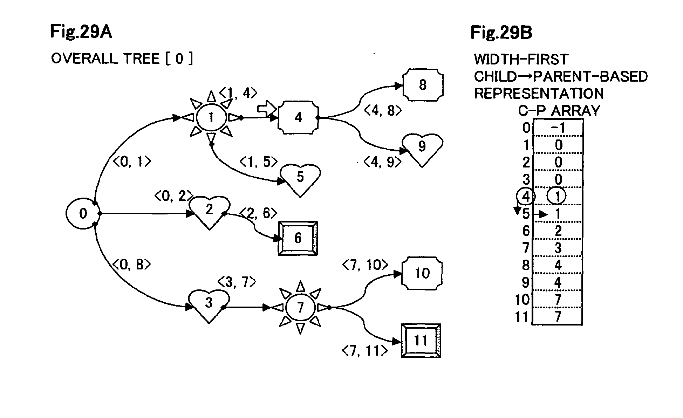

It is now assumed that, in Fig. 29A, a vertex node list is represented by [4, 5, 6, 7]. The vertex node having the node identifier "4" (see the arrow) is now focused on. Then, it is seen that the value corresponding to the node identifier "4" in the C-P array is "1" and the value corresponding to the node identifier "5" in the C-P array is also "1". Accordingly, since both values are equal to each other, the node identifier "5" is stored in a new vertex node list. Concerning the node identifiers "5", "6", and "7", the values corresponding to the node identifiers in the C-P array are different from the values obtained by adding one to the values corresponding to the node identifiers in the C-P array. Accordingly, these vertex nodes disappear. Thus, the new vertex node list can be represented by [5].

When a vertex node is moved to a node corresponding to an "older sibling", the value in the C-P array indicated by the node identifier in the vertex node list is compared with the value in the C-P array indicated by the node identifier one before the node identifier of the vertex node (i.e., node identifier having the value obtained by subtracting one from the node identifier of the vertex node).

### [Processing Executed when Moving Vertex Node (Depth-first Mode)]

Processing executed when moving a vertex node according to an embodiment of the present invention is discussed below. A description is first given of the movement of a vertex node when an array (C-P array) based on "child→parent" representation created from a tree-type data structure based on a depth-first mode is used.

In the depth-first mode, processing executed by the computer system 10 when moving a vertex node to a node corresponding to a child is similar to that shown in Fig. 24. In the C-P array in the depth-first mode, however, values are not arranged in ascending order. The nodes corresponding to children appear in a range of nodes starting from the node having the node identifier subsequent to the node identifier of the vertex node in the vertex node list (in this embodiment, the node identifier obtained by adding one to the node identifier of the vertex node) to the node identifier one before the node identifier whose value in the C-P array indicated by the node identifier becomes smaller than the node identifier of the vertex node in the vertex node list (in this embodiment, the node identifier smaller than the node identifier of the vertex node by "1").

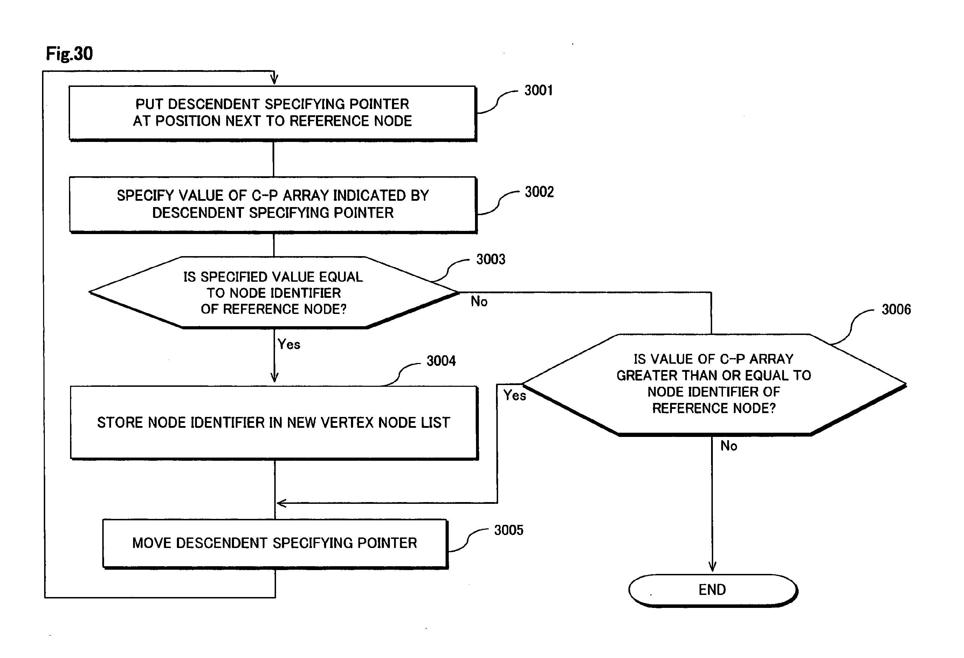

Accordingly, when searching for a node corresponding to a child, as shown in Fig. 30, on the basis of a reference node, which is a node in a vertex node list, the computer system 10 puts a search pointer at a position subsequent to the reference node (i.e., the position indicated by the node identifier obtained by adding "1" to the node identifier of the reference node) (step 3001), and specifies the value in the C-P array indicated by the search pointer (step 3002). Then, the computer system 10 determines whether the specified value is the same as the node identifier of the reference node (step 3003). If the result of step 3003 is YES, the computer system 10 stores the node identifier at which the search pointer is positioned in a new vertex node list (step 3004). Then, the search pointer is allowed to advance by one (step 3005).

If it is determined that the result of step 3003 is NO, it is determined whether the value in the C-P array is greater than or equal to the node identifier of the reference node (step 3006). If the result of step 3006 is YES, the search pointer is allowed to advance by one for subsequent processing since the node having the node identifier at which the search pointer is positioned is a descendent of the reference node (step 3005). If the result of step 3006 is NO, it means that the node having the node identifier at which the search pointer is positioned is not a descendent of the vertex node, and thus, the processing is terminated.

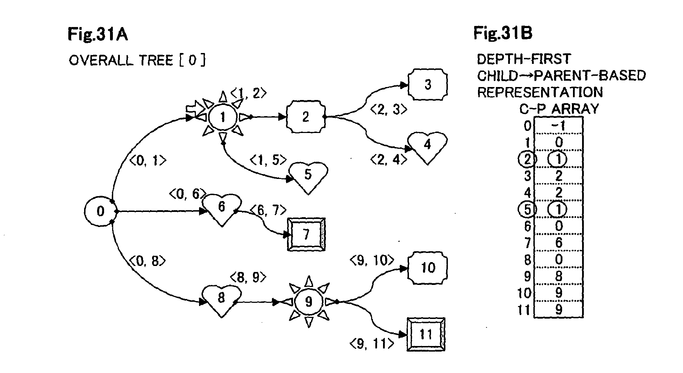

It is now assumed that, in the example shown in Fig. 31A, the vertex node list is represented by [1, 6, 8]. The vertex node having the node identifier "1" (see the arrow) is now focused on. Then, the search pointer is initially disposed at the position of the node identifier "2". At this point, since the value in the C-P array indicated by the search pointer is "1", the node identifier "2" is stored in a new vertex node list.

When the search pointer is positioned at the node identifier "5", the value in the C-P array indicated by the search pointer is "1". Accordingly, the node identifier "5" is stored in the new vertex node list. Then, when the search pointer is positioned at the node identifier "6", the value in the C-P array indicated by the search pointer is "0", which is smaller than the node identifier "1" of the reference node, and thus, the processing is terminated.

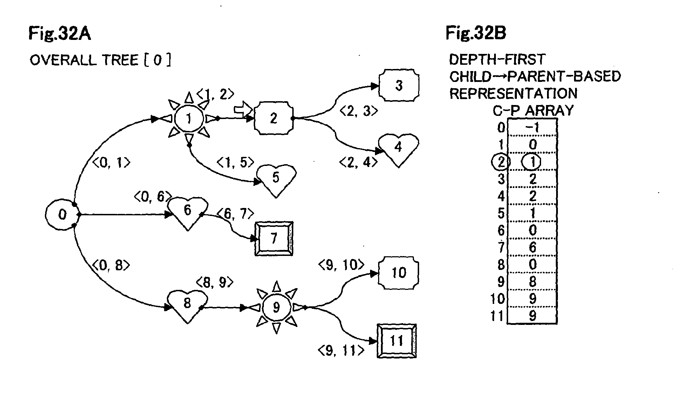

The processing executed by the computer system 10 when moving a vertex node to a node corresponding to a parent is now described below. The processing executed when moving a vertex node to a node corresponding to a parent is similar to that shown in Fig. 26. It is now assumed that, in the example shown in Fig. 32A, the vertex node list is represented by [2, 5, 7, 9]. The vertex node having the node identifier "2" (see the arrow) is now focused on. It can be seen that the value in the C-P array (see Fig. 32B) corresponding to the node identifier "2" is "1", and the value "1" is stored in a new vertex node list. Similarly, concerning the node identifiers "5", "7", and "9", the values in the C-P array corresponding to the node identifiers are "1", "6", and "8", respectively, and thus, the new vertex node list can be represented by [1, 1, 6, 8].

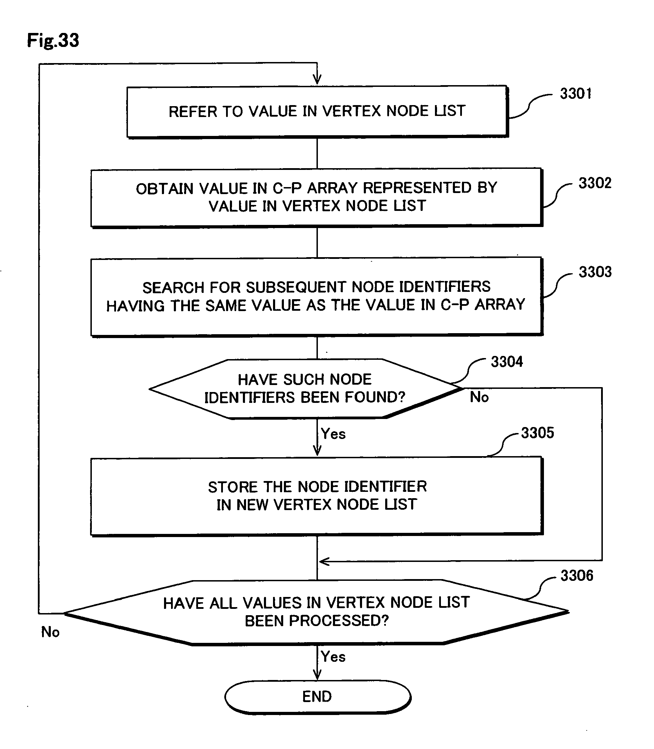

Fig. 33 is a flowchart illustrating processing executed by the computer system 10 when moving a vertex node to a node corresponding to a younger sibling. As shown in Fig. 33, the computer system 10 refers to a value in a vertex node list (step 3301), and obtains the value (node identifier) in the C-P array indicated by the value in the vertex node list (step 3302). Then, the computer system 10 searches for, among subsequent node identifiers (after the node identifiers greater than the above-described node identifier by "1"), a node identifier having the same value in the C-P array (step 3303). If the node identifier having the same value in the C-P array is found (YES in step 3304), the computer system 10 stores the node identifier having the same value in a new vertex node (step 3305). If such a node identifier having the same value is not found, it is determined that the vertex node has disappeared.

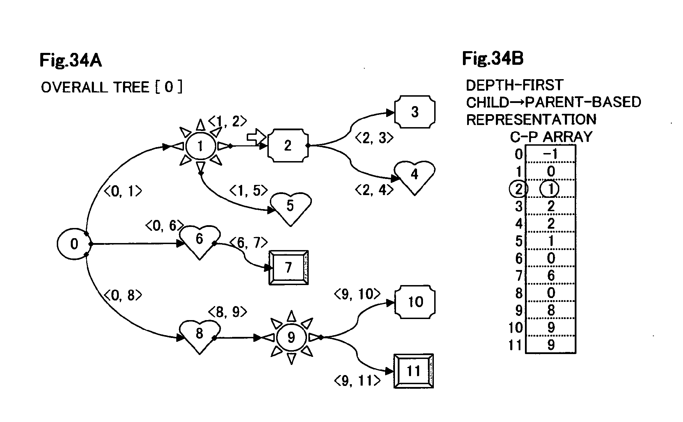

The computer system executes steps 3301 through 3305 on all the values in the vertex node list (step 3306), and then, the node identifiers of the new vertex nodes corresponding to younger siblings are stored in the new vertex node list. It is now assumed that, in Fig. 34A, the vertex node list is represented by [2, 5, 7, 9]. The vertex node having the node identifier "2" is now focused on. Then, it can be seen that the value in the C-P array corresponding to the node identifier is "1". According to the processing shown in Fig. 33, the node identifier "5" having the value "1" in the C-P array can be searched, and the node identifier "5" is stored in the vertex node list. Concerning the node identifiers "5", "7", and "9", the same value cannot be found from the C-P array. That is, nodes having the same number as the parent node cannot be found.
Accordingly, these vertex nodes disappear after being moved. As a result, the vertex node list of the new vertex nodes can be represented by "5".

### [Information Processing Apparatus]

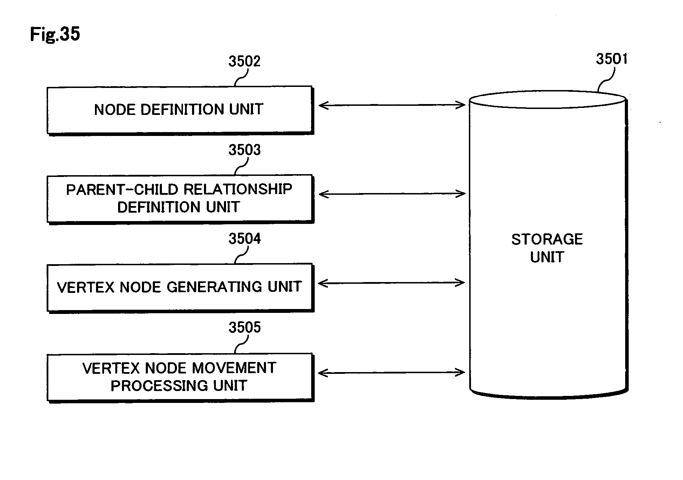

Fig. 35 is a functional block diagram illustrating an information processing apparatus 3500 according to an embodiment of the present invention for constructing a tree-type data structure and for generating a vertex node list and also generating a vertex node list after vertex nodes are moved. The information processing apparatus 3500 is implemented by installing a required program into the computer system 10 shown in Fig. 1.

The information processing apparatus 3500 includes, as shown in Fig. 35, a storage unit 3501 storing data representing a tree-type data structure and a vertex node list, a node definition unit 3502 for assigning unique node identifiers to nodes including the root node and for storing the node identifiers in the storage unit 3501, a parent-child relationship definition unit 3503 for associating the node identifiers assigned to the non-root nodes, which are nodes other than the root node, with the node identifiers assigned to the parent nodes of the non-root nodes and for storing a C-P array, which is an array indicating the association between the identifiers of the non-root nodes and the identifiers of the parent nodes of the non-root nodes, in the storage unit 3501, a vertex node list generating unit 3504 for generating a vertex node list on the basis of the node identifiers and the C-P array stored in the storage unit 3501, and a vertex node movement processing unit 3505 for moving vertex nodes in response to, for example, an instruction from an input unit (see reference numeral 24 in Fig. 1) and for generating a new vertex node list indicating moved versions of the vertex nodes. The vertex node list generated by the vertex node list generating unit 35 and the new vertex node list generated by the vertex node movement processing unit 3505 are stored in the storage unit 3501.

Preferably, the node definition unit 3502 uses numerical values as the node identifiers, and more preferably, uses serial integers as the node identifiers. The parent-child relationship definition unit 3503 stores an array including sets of the node identifiers assigned to the non-root nodes and the node identifiers assigned to the associated parent nodes in the storage unit 3501.

When a node is specified in response to, for example, an instruction from the input unit (see reference numeral 24 in Fig. 1), the vertex node list generating unit 3504 stores the node identifier of the specified node in the vertex node list. When an instruction to move a vertex node (to a node corresponding to a parent, a child, a younger sibling, or an older sibling) is given from, for example, the input unit, the vertex node movement processing unit 3305 obtains the C-P array and the vertex node list from the storage unit 3501 to generate a new vertex node list and stores it in the storage unit 3501.

The present invention is not limited to the disclosed exemplary embodiments. Various modifications may be made within the scope of the following claims, and it is needless to say that those modifications are encompassed in the scope of the invention.

### Brief Description of the Drawings

[Fig. 1] Fig. 1 is a block diagram illustrating a computer system that handles a tree-type data structure according to an embodiment of the present invention.
[Fig. 2] Figs. 2A and 2B illustrate POS data, which is an example of tree-type data: Fig. 2 illustrates an example of a visual representation of the data structure (i.e., topology) and the data values of this tree-type data; and Fig. 2B illustrates an example of the same tree-type data represented as an XML format.
[Fig. 3] Figs. 3A through 3C illustrate one example of representation of a tree-type data structure using an arclist.
[Fig. 4] Figs. 4A through 4C illustrate a representation method for a tree-type data structure based on a "child→parent" relationship according to an embodiment of the present invention.
[Fig. 5] Fig. 5 is a flowchart illustrating a method for constructing a tree-type data structure on a storage device according to an embodiment of the present invention.
[Fig. 6] Figs. 6A through 6C illustrate processing for converting ID-based tree-structured data into serial-integer-based tree-structured data according to an embodiment of the present invention.
[Fig. 7] Figs. 7A through 7C illustrate processing for converting ID-based tree-structured data into serial-integer-based tree-structured data according to another embodiment of the present invention.
[Fig. 8] Fig. 8 is a flowchart illustrating depth-first-based node definition processing according to an embodiment of the present invention.
[Fig. 9] Fig. 9 illustrates a parent-child relationship array based on "child→parent" representation created according to an embodiment of the present invention.
[Fig. 10] Fig. 10 illustrates a parent-child relationship array based on "parent→child" representation and created from a depth-first tree-type data structure shown in Fig. 6C.
[Fig. 11] Fig. 11 is a flowchart illustrating width-first-based node definition processing according to an embodiment of the present invention.
[Fig. 12] Fig. 12 illustrates a parent-child relationship array based on "child→parent" representation created according to an embodiment of the present invention.
[Fig. 13] Fig. 13 illustrates a parent-child relationship array based on "parent→child" representation and created from a width-first tree-type data structure shown in Fig. 7C.
[Fig. 14] Fig. 14A illustrates a width-first-based tree-type data structure, and Fig. 14B illustrates the tree data structure as a parent-child relationship array based on "child→parent" representation.
[Fig. 15] Fig. 15A illustrates an example of a vertex node list, and Fig. 15B illustrates an example of a partial tree group specified by the vertex node list.
[Fig. 16] Fig. 16A illustrates an example of a vertex node list obtained by search processing, and Fig. 16B illustrates an example of a partial tree group specified by the vertex node list.
[Fig. 17] Fig. 17A illustrates an example of an array indicating a vertex node list and an array indicating a counting result obtained by counting processing, and Fig. 17B illustrates an example of a partial tree group specified by the vertex node list.
[Fig. 18] Fig. 18A illustrates an example of an array indicating a vertex node list sorted by the number of nodes and an array indicating the associated number of nodes, and Fig. 18B illustrates an example of partial trees specified by the vertex node list.
[Fig. 19] Figs. 19A and 19C illustrate examples of vertex node lists to be subjected to a logical AND operation, and Figs. 19B and 19D illustrate examples of partial tree groups specified by the vertex node lists shown in Figs. 19A and 19C, respectively.
[Fig. 20] Fig. 20A illustrates an example of a vertex node list indicating the result of a logical AND operation, and Fig. 20B illustrates a partial tree group specified by the vertex node list.
[Fig. 21] Fig. 21A illustrates a vertex node list according to this embodiment, Fig. 21B illustrates a tree in which vertex nodes are pointed by the arrows, Fig. 21C illustrates a vertex node list after vertex nodes are moved to nodes corresponding to children, and Fig. 21D illustrates an example of a tree in which moved versions of the vertex nodes are indicated by the arrows.
[Fig. 22] Fig. 22A illustrates a vertex node list according to this embodiment, Fig. 22B illustrates a tree in which vertex nodes are pointed by the arrows, Fig. 22C illustrates a vertex node list after vertex nodes are moved to nodes corresponding to parents, and Fig. 22D illustrates an example of a tree in which moved versions of the vertex nodes are indicated by the arrows.
[Fig. 23] Fig. 23A illustrates a vertex node list according to this embodiment, Fig. 23B illustrates a tree in which vertex nodes are pointed by the arrows, Fig. 23C illustrates a vertex node list after vertex nodes are moved to nodes corresponding to younger siblings, and Fig. 23D illustrates an example of a tree in which moved versions of the vertex nodes are indicated by the arrows.
[Fig. 24] Fig. 24 is a flowchart illustrating processing executed by a computer system when moving vertex nodes to nodes corresponding to children.
[Fig. 25] Fig. 25A illustrates an example of a tree, and Fig. 25B illustrates processing for moving vertex nodes to nodes corresponding to children.
[Fig. 26] Fig. 26 is a flowchart illustrating processing executed by the computer system when moving vertex nodes to nodes corresponding to parents.
[Fig. 27] Fig. 27A illustrates an example of a tree, and Fig. 27B illustrates processing for moving vertex nodes to nodes corresponding to parents.
[Fig. 28] Fig. 28 is a flowchart illustrating processing executed by the computer system when moving vertex nodes to nodes corresponding to younger siblings.
[Fig. 29] Fig. 29A illustrates an example of a tree, and Fig. 29B illustrates processing for moving vertex nodes to nodes corresponding to younger siblings.
[Fig. 30] Fig. 30 is a flowchart illustrating processing executed by the computer system when searching for nodes corresponding to children.
[Fig. 31] Fig. 31A illustrates an example of a tree, and Fig. 31B illustrates processing for moving vertex nodes to nodes corresponding to children.
[Fig. 32] Fig. 32A illustrates an example of a tree, and Fig. 32B illustrates processing for moving vertex nodes to nodes corresponding to parents.
[Fig. 33] Fig. 33 is a flowchart illustrating processing executed by the computer system when moving vertex nodes to nodes corresponding to younger siblings.
[Fig. 34] Fig. 34A illustrates an example of a tree, and Fig. 34B illustrates processing for moving vertex nodes to nodes corresponding to younger siblings.
[Fig. 35] Fig. 35 is a functional block diagram illustrating an information processing apparatus for constructing a tree-type data structure according to this embodiment and a vertex node list on a storage device.

### Reference Numerals

- 10: computer system
- 12: CPU
- 14: RAM
- 16: ROM
- 18: fixed storage device
- 20: CD-ROM driver
- 22: I/F
- 24: input device
- 26: display device
- 3500: information processing apparatus
- 3501: storage Unit
- 3502: node definition unit
- 3503: parent-child relationship definition unit
- 3504: vertex node generating unit
- 3505: vertex node movement processing unit

## Claims

1. An array generation method, in a computer including data having a tree-type data structure in which unique node identifiers are assigned to nodes and a parent-child relationship between the nodes is represented by a first array including pairs, each pair being formed of a node identifier assigned to each of non-root nodes, which are nodes other than a root node, and a node identifier of a parent node with which each of the non-root nodes is associated, the array generation method comprising:
a step of providing a second array, in order to represent at least one node group, each including a specific node and a descendent node of the specific node, the second array storing node identifiers of the specific nodes, which serve as vertex nodes; and
a step of generating a third array storing node identifiers of new vertex nodes, which are moved versions of the vertex nodes whose node identifiers are stored in the second array, by referring to the first array, wherein each of the vertex nodes is moved to one of
a) a child node directly connected to the vertex node by an arc which is extended from the vertex node to the child node,
b) a parent node directly connected to the vertex node by an arc which is extended from the parent node to the vertex node,
c) an older sibling node which is in the same generation as the vertex node, an arc from the parent node of the vertex node being connected to the older sibling node before another arc from the parent node of the vertex node is connected to the vertex node, and
d) a younger sibling node which is in the same generation as the vertex node, an arc from the parent node of the vertex node being connected to the younger sibling node after another arc from the parent node of the vertex node is connected to the vertex node.

2. The method according to claim 1, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to a child node includes
a step of specifying, in the first array, a storage location at which the node identifier of the vertex node is stored, and
a step of determining a node identifier of a moved version of the vertex node to be a node identifier corresponding to the storage location.

3. The method according to claim 1, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to a parent node includes
a step of specifying, in the first array, a node identifier stored at a location corresponding to the node identifier of the vertex node, and
a step of determining a node identifier of a moved version of the vertex node to be the node identifier stored at the corresponding location.

4. The method according to claim 1, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to an older sibling node includes
a step of specifying, in the first array, a first node identifier stored at a location corresponding to the node identifier of the vertex node,
a step of specifying, in the first array, a second node identifier stored at a storage location having a value smaller than a value of the location corresponding to the node identifier of the vertex node by one, and
a step of determining, when the first node identifier and the second node identifier coincide with each other, a node identifier of a moved version of the vertex node to be a node identifier corresponding to the storage location at which the second node identifier is stored.

5. The method according to claim 1, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to a younger sibling node includes
a step of specifying, in the first array, a first node identifier stored at a location corresponding to the node identifier of the vertex node,
a step of specifying, in the first array, a third node identifier stored at a storage location having a value greater than a value of the location corresponding to the node identifier of the vertex node by one, and
a step of determining, when the first node identifier and the third node identifier coincide with each other, a node identifier of a moved version of the vertex node to be a node identifier corresponding to the storage location at which the third node identifier is stored.

6. The method according to claim 1, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to a child node includes
a step of specifying, in the first array, a storage location at which the node identifier of the vertex node is stored, and
a step of determining a node identifier of a moved version of the vertex node to be a node identifier corresponding to the storage location.

7. The method according to claim 1, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to a parent node includes
a step of specifying, in the first array, a node identifier stored at a location corresponding to the node identifier of the vertex node, and
a step of determining a node identifier of a moved version of the vertex node to be the node identifier stored at the corresponding location.

8. The method according to claim 1, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to an older sibling node includes
a step of specifying, in the first array, a first node identifier stored at a storage location at which the node identifier of the vertex node is stored,
a step of searching, in the first array, a fourth node identifier stored at storage locations having values smaller than a value of the storage location at which the node identifier of the vertex node is stored, the fourth identifier being equal to the first identifier,
a step of specifying a storage location having a largest value among the storage locations of the fourth node identifier, and
a step of determining a node identifier of a moved version of the vertex node to be a node identifier corresponding to the storage location having the largest value.

9. The method according to claim 1, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
the step of generating the third array for moving each of the vertex nodes to a younger sibling node includes
a step of specifying, in the first array, a first node identifier stored at a storage location at which the node identifier of the vertex node is stored,
a step of searching, in the first array, a fifth node identifier stored at storage locations having values greater than a value of the storage location at which the node identifier of the vertex node is stored, the fifth node identifier being equal to the first node identifier,
a step of specifying a storage location having a smallest value among the storage locations of the fifth node identifier, and
a step of determining a node identifier of a moved version of the vertex node to be a node identifier corresponding to the storage location having the largest value.

10. An array generation program readable by a computer which includes data having a tree-type data structure, in which unique node identifiers are assigned to nodes and a parent-child relationship between the nodes is represented by a first array including pairs, each pair being formed of a node identifier assigned to each of non-root nodes, which are nodes other than a root node, and a node identifier of a parent node with which each of the non-root nodes is associated, the array generation program allowing the computer to execute:
a step of providing a second array, in order to represent at least one node group, each including a specific node and a descendent node of the specific node, the second array storing node identifiers of the specific nodes, which serve as vertex nodes; and
a step of generating a third array storing node identifiers of new vertex nodes, which are moved versions of the vertex nodes whose node identifiers are stored in the second array, by referring to the first array, wherein each of the vertex nodes is moved to one of
a) a child node directly connected to the vertex node by an arc which is extended from the vertex node to the child node,
b) a parent node directly connected to the vertex node by an arc which is extended from the parent node to the vertex node,
c) an older sibling node which is in the same generation as the vertex node, an arc from the parent node of the vertex node being connected to the older sibling node before another arc from the parent node of the vertex node is connected to the vertex node, and
d) a younger sibling node which is in the same generation as the vertex node, an arc from the parent node of the vertex node being connected to the younger sibling node after another arc from the parent node of the vertex node is connected to the vertex node.

11. The program according to claim 10, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to a child node, the program allows the computer to execute
a step of specifying, in the first array, a storage location at which the node identifier of the vertex node is stored, and
a step of determining a node identifier of a moved version of the vertex node to be a node identifier corresponding to the storage location.

12. The program according to claim 10, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to a parent node, the program allows the computer to execute
a step of specifying, in the first array, a node identifier stored at a location corresponding to the node identifier of the vertex node, and
a step of determining a node identifier of a moved version of the vertex node to be the node identifier stored at the corresponding location.

13. The program according to claim 10, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to an older sibling node, the program allows the computer to execute
a step of specifying, in the first array, a first node identifier stored at a location corresponding to the node identifier of the vertex node,
a step of specifying, in the first array, a second node identifier stored at a storage location having a value smaller than a value of the location corresponding to the node identifier of the vertex node by one, and
a step of determining, when the first node identifier and the second node identifier coincide with each other, a node identifier of a moved version of the vertex node to be a node identifier corresponding to the storage location at which the second node identifier is stored.

14. The program according to claim 10, wherein:
unique serial integers are assigned to the nodes including the root node by giving priority to nodes in the same generation as a certain node rather than child nodes of that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to a younger sibling node, the program allows the computer to execute
a step of specifying, in the first array, a first node identifier stored at a location corresponding to the node identifier of the vertex node,
a step of specifying, in the first array, a third node identifier stored at a storage location having a value greater than a value of the location corresponding to the node identifier of the vertex node by one, and
a step of determining, when the first node identifier and the third node identifier coincide with each other, a node identifier of a moved version of the vertex node to be a node identifier corresponding to the storage location at which the third node identifier is stored.

15. The program according to claim 10, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to a child node, the program allows the computer to execute
a step of specifying, in the first array, a storage location at which the node identifier of the vertex node is stored, and
a step of determining a node identifier of a moved version of the vertex node to be a node identifier corresponding to the storage location.

16. The program according to claim 10, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to a parent node, the program allows the computer to execute
a step of specifying, in the first array, a node identifier stored at a location corresponding to the node identifier of the vertex node, and
a step of determining a node identifier of a moved version of the vertex node to be the node identifier stored at the corresponding location.

17. The program according to claim 10, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to an older sibling node, the program allows the computer to execute
a step of specifying, in the first array, a first node identifier stored at a storage location at which the node identifier of the vertex node is stored,
a step of searching, in the first array, a fourth node identifier stored at storage locations having values smaller than a value of the storage location at which the node identifier of the vertex node is stored, the fourth identifier being equal to the first identifier,
a step of specifying a storage location having a largest value among the storage locations of the fourth node identifier, and
a step of determining a node identifier of a moved version of the vertex node to be a node identifier corresponding to the storage location having the largest value.

18. The program according to claim 10, wherein:
unique serial integers are assigned to the nodes including the root node by giving priority to child nodes of a certain node rather than nodes in the same generation as that certain node;
the first array is formed by arranging the integers assigned to the parent nodes of the corresponding non-root nodes, which are nodes other than the root node, according to an order in which the integers are assigned to the non-root nodes; and
in the step of generating the third array for moving each of the vertex nodes to a younger sibling node, the program allows the computer to execute
a step of specifying, in the first array, a first node identifier stored at a storage location at which the node identifier of the vertex node is stored,
a step of searching, in the first array, a fifth node identifier stored at storage locations having values greater than a value of the storage location at which the node identifier of the vertex node is stored, the fifth node identifier being equal to the first node identifier,
a step of specifying a storage location having a smallest value among the storage locations of the fifth node identifier, and
a step of determining a node identifier of a moved version of the vertex node to be a node identifier corresponding to the storage location having the largest value.
